# EUROPÄISCHE PATENTSCHRIFT

(11) **EP 0 864 656 B1**
(45) Veröffentlichungstag und Bekanntmachung des Hinweises auf die Patenterteilung: **02.11.2005**
(21) Anmeldenummer: 98103701.3
(22) Anmeldetag: 03.03.1998
(51) Int. Cl.: C12Q 1/68, C12N 15/63

(54) **In vitro Transkriptionsverfahren zum Screening von Naturstoffen und anderen chemischen Substanzen**
Method of in vitro transcription for the screening for natural products and other chemical substances
Méthode de transcription in vitro pour la détection des produits naturels et d'autres substances chimiques

(30) Priorität: 12.03.1997 DE 19710159
(43) Veröffentlichungstag der Anmeldung: 16.09.1998
(73) Patentinhaber: Sanofi-Aventis Deutschland GmbH, 65929 Frankfurt am Main (DE)
(72) Erfinder: Kirschbaum, Bernd, Dr., 55122 Mainz (DE); Stahl, Wilhelm, Dr., 65510 Idstein (DE); Winkler, Irvin, Dr., 65835 Liederbach (DE); Meisterernst, Michael, Dr., 82223 Eichenau (DE)

(56) Entgegenhaltungen:
- SAWADOGO AND ROEDER: "FACTORS INVOLVED IN SPECIFIC TRANSCRIPTION BY HUMAN RNA POLYMERASE II: ANALYSIS BY A RAPID AND QUANTITATIVE IN VITRO ASSAY" PROCEEDINGS OF THE NATIONAL ACADEMY OF SCIENCES OF USA, NATIONAL ACADEMY OF SCIENCE. WASHINGTON, US, Bd. 82, Juli 1985 (1985-07), Seiten 4394-4398, XP002118950 ISSN: 0027-8424 & DIGNAM J. ET AL.: "ACCURATE TRANSCRIPTION INITIATION BY RNA POLUMERASE II IN A SOLUBLE EXTRACT FROM ISOLATED MAMMALIAN NUCLEI" NAR, Bd. 11, Nr. 5, 1983, Seiten 1475-1489, XP002006584
- ANNWEILER ARND ET AL: "Analysis of transcriptional stimulation by recombinant Oct proteins in a cell-free system." JOURNAL OF BIOLOGICAL CHEMISTRY, Bd. 268, Nr. 4, 1993, Seiten 2525-2534, XP002206216 ISSN: 0021-9258
- PAO CHING-I ET AL: "In vitro transcription of the rat insulin-like growth factor-I gene." JOURNAL OF BIOLOGICAL CHEMISTRY, Bd. 271, Nr. 15, 1996, Seiten 8667-8674, XP002206217 ISSN: 0021-9258
- WEN L-P ET AL: "DIOXIN-INDUCIBLE AH RECEPTOR-DEPENDENT TRANSCRIPTION IN-VITRO" PROCEEDINGS OF THE NATIONAL ACADEMY OF SCIENCES OF THE UNITED STATES, Bd. 87, Nr. 21, 1990, Seiten 8545-8549, XP002206218 1990 ISSN: 0027-8424

## Beschreibung

Die Erfindung betrifft ein automatisierbares in vitro Verfahren zur Analyse der Transkription viraler und zellulärer Gene, welches für ein Massenscreening zur Auffindung spezifischer, die Genaktivität selektiv beeinflussender chemischer Leitstrukturen in einer rationellen und ökonomisch vertretbaren Weise geeignet ist.

Das Screening von Naturstoffen auf biologisch aktive Inhaltsstoffe erhielt neuen Aufschwung, nachdem sich gezeigt hatte, daß mit rationalem Wirkstoffdesign allein keine erfolgreiche Wirkstoffsuche möglich ist. So stehen neben chemischen Substanzbibliotheken und kombinatorischen Bibliotheken erneut klassische Naturstoffextrakte als Substanzquellen im Mittelpunkt des Interesses. Dies liegt vor allem an der Vielfalt der in diesen Extrakten enthaltenen Substanzen. Mit modernen analytischen Methoden läßt sich nachweisen, daß Extrakte mikrobieller Fermentation etwa 500 strukturell stark unterschiedliche Verbindungsklassen enthalten. Damit sind sie hinsichtlich ihrer Diversität chemischen und kombinatorischen Substanzbibliotheken weit überlegen.

Limitierend für die pharmakologische Nutzung des reichhaltigen und bisher weitgehend unerforschten Potentials von Naturstoffen ist die Anzahl an kompatiblen, aussagekräftigen Verfahren, mit denen potentielle Wirkstoffe getestet werden können. Insbesondere werden Verfahren benötigt, die zur Identifizierung von hochspezifischen pharmakologischen Wirkstoffen, deren Applikation mit möglichst geringen Nebenwirkungen verbunden ist, eingesetzt werden können.

Das im folgenden beschriebene Verfahren beruht auf einem Ansatz, bei dem Substanzen auf ihr Potential getestet werden, bereits in den ersten Schritt der Umsetzung genetischer Information, die Regulation der Transkription von Genen, einzugreifen. Mit einem solchen Verfahren sollen Substanzen identifiziert werden, die die Transkription direkt oder indirekt positiv oder negativ beeinflussen können.

Die Stärke der Transkription eines Gens wird durch die genregulatorischen Elemente dieses Gens, insbesondere durch den Promotor, durch Enhancer oder Silencer bestimmt. Die Wirkung der genregulatorischen Elemente wird durch Transkriptions- und Kofaktoren vermittelt und umgesetzt. Diese Transkriptionsfaktoren können die Transkriptionsrate eines Gens sowohl negativ als auch positiv beinflussen und tragen dadurch zur Stärke der Transkription bei. Mittlerweile wurden eine Vielzahl von Transkriptionsfaktoren als wichtige "molekulare Schalter" im Verlauf vieler zellulärer Vorgänge, einschließlich Signaltransduktion, Zellzykluskontrolle, Differenzierung und kontrolliertem Zelltod (Apoptose) identifiziert.

Die meisten von der Zelle empfangenen Signale, die die Stärke der Transkription von Genen beinflussen, werden von Transmembranproteinen "registriert", intrazellulär durch Signaltransduktionsketten weitergeleitet und von Transkriptionsfaktoren umgesetzt. Beispiele für Proteine, die Außensignale empfangen, sind cAMP-bindende Proteine, Sensoren für Wachstumssignale (wie der Serum Response Faktor, SRF), Hormonrezeptoren oder Transkriptionsfaktoren, die an der Expression von Zytokinen beteiligt sind, sogenannte STAT Proteine (signal transducers and activators of transcription).

Mittlerweile sind eine ganze Reihe von Substanzen bekannt, die direkt oder indirekt die Stärke der Transkription von Genen beeinflussen. Solche Substanzen werden u.a. als pharmakologische Wirkstoffe in Arzneimitteln eingesetzt, obwohl die Wirkung dieser Substanzen oft nicht spezifisch ist. Deshalb ist die Einnahme solcher Arzneimittel auch häufig mit unerwünschten Nebenwirkungen verbunden.

Beispielsweise werden zur Behandlung von immunologischen Erkrankungen Arzneimittel eingesetzt, die Cyclosporin- und Steroidderivate als Wirkstoffe enthalten. Cyclosporin A bildet einen Komplex mit Cyclophilin. Dieser inhibhiert Calcineurin, eine ubiquitäre Phosphatase, die Proteine in unterschiedlichen Stoffwechselwegen dephosphoryliert. Calcineurin reguliert z.B. die Verlagerung einer Untereinheit des Transkriptionsfaktors NFAT vom Zytosol in den Zellkem (Liu, J. (1993) Immunology Today 14, 290-295). NFAT (nuclear factor of activated T-cells) ist an der Aktivierung einiger immunologisch relevanter Gene beteiligt. Cyclosporin A (CsA) reguliert indirekt über die Beeinflußung von NFAT (nuclear factor of activated T-cells) die Expression dieser Gene. Da Cyclosporin A aber nur indirekt, nämlich über das ubiquitäre Calcineurin, die Aktivität von NFAT reguliert, wirkt Cyclosporin A über andere Stoffwechselwege auch gefäßverengend sowie nephro- und neurotoxisch. Wäre ein pharmakologischer Wirkstoff bekannt, mit dem NFAT spezifisch, möglicherweise direkt gehemmt werden könnte, so würde ein Medikament das diesen Wirkstoff enthält, voraussichtlich weniger Nebenwirkungen hervorrufen.

Auch Glukokortikoide zählen zu den pharmakologischen Wirkstoffen, die neben der gewünschten Wirkung auch mit starken Nebenwirkungen verbunden sind. Glukokortikoide werden seit vielen Jahren zur Standardtherapie bei Allergien, Rheuma, Entzündungen und anderen Erkrankungen, die auf ein überreaktives Immunsystem zurückzuführen sind, eingesetzt. Sie bewirken unter anderem die Hemmung der Aktivierung des zelltypspezifischen Transkriptionsfaktors NFκB (Scheinmann, R.I., Cogswell, P.C., Lofquist, A.K. & Baldwin Jr., A.S. (1995) Science 270, 283-286; Auphan, N., DiDonato, J.A., Rosette, C., Helmberg, A. & Karin M. (1995) Science 270, 286-290), indem sie die Bildung eines zellulären Inhibitors von NFκB, dem IκB-Protein stimulieren. IκB wiederum verhindert den Transfer aktiver NFκB-Dimere in den Zellkern und somit die Aktivierung wichtiger immunologischer Zielgene. Der Einfluß von Glukokortikoiden auf die Genexpression ist ähnlich wie im Fall von CsA relativ unspezifisch, da Glukokortikoide nicht nur auf NFκB, sondern auch auf andere Proteine wirken.

Diese Beispiele verdeutlichen, daß ein großer Bedarf an pharmakologischen Wirkstoffen besteht, die ein möglichst spezifisches Wirkprofil aufweisen. Um neue chemische Leitstrukturen mit entsprechenden Eigenschaften aufzufinden, müssen eine Vielzahl von Substanzen auf ihre spezifische Wirksamkeit getestet werden.

Trotz identischer Gen-Ausstattung werden in Abhängigkeit vom Zelltyp und/oder bestimmten Erkrankungen oder Defekten sowie dem jeweiligen Entwicklungs- und Differenzierungsgrad einzelner Zellen immer nur bestimmte Proteine exprimiert. Als Grundlage dieser Individualität von Zellen kann das spezifische Repertoire genregulatorischer Proteine angesehen werden, beispielsweise die zelltyp- und entwicklungsspezifische Ausstattung mit bestimmten Transkriptions- und Kofaktoren (accessorischen Proteinen), die die koordinierte und kontrollierte Transkription distinkter Gene regulieren.

Spezifische pharmakologische Wirkstoffe sollten deshalb selektiv die Transkription pathologisch relevanter Gene in Zellen eines definierten Typs aktivieren oder inhibieren. Um solche Wirkstoffe zu identifizieren, wird ein Transkriptionsverfahren benötigt, bei dem der Effekt von zu testenden Wirkstoffen auf die Transkription einzelner Gene, d.h. auf die an der Regulation der Transkription beteiligten Proteine und auf die genregulatorischen Elemente unter definierten Bedingungen direkt messbar ist. Da eine Vielzahl von potentiellen Wirkstoffen getestet werden müssen, sollte das Verfahren darüber hinaus einfach durchzuführen und automatisierbar sein.

Das erste zellfreie Transkriptionsverfahren wurde von Weil et al. beschrieben (Weil, P.A., Luse, D.S., Segall, J., Roeder, R.G. (1979) Cell 18, 469-484). Dabei wurden angereicherte Extrakte aus Zellkernen, sogenannte S100-Extrakte (Weil, P.A., Segall, J., Harris, B. Ng, S.Y., Roeder, R.G. (1979) J. Biol. Chem. 254, 6163-6173) und gereinigte RNA-Polymerase II für die in vitro Transkription eingesetzt. Ohne exogene RNA Polymerase II waren diese angereicherten, aber nicht weiter aufgereinigten Kernextrakte nicht transkriptionsfähig (Weil, P.A., Luse, D.S., Segall, J., Roeder, R.G. (1979) Cell 18, 469-484; Dignam, J.D., Martin, P.L., Shastry, B.S., Roeder, R.G. (1983) Methods in Enzymology 101, 582-598).

Ausgehend von solchen Kernextrakten wurden anschließend Verfahren entwickelt, mit denen über mehrstufige Reinigungsschritte Transkriptionsfaktoren isoliert werden können. Unter anderem beinhalten diese Verfahren Reinigungsschritte, bei denen die Kernextrakte chromatographisch über kernproteinbindende Materialien, wie z.B. über Phosphocellulosesäulen, gereinigt werden. Dignam et al. haben im Rahmen dieser aufwendigen, mehrstufigen Verfahren für einen der Reinigungsschritte erstmals die Verwendung des käuflichen P11-Systems® (Whatman, Maidstone, England) beschrieben (Dignam, J.D., Martin, P.L., Shastry, B.S., Roeder, R.G. (1983) Methods in Enzymology 101, 582-598).

Die mehrstufigen Aufreinigungsverfahren wurden immer mehr angepaßt, so daß es mittlerweile möglich ist, aus den Extrakten von Zellkernen über aufwendige Verfahren einzelne Transkriptionsfaktoren zu isolieren. Darüber hinaus sind einzelne Faktoren beziehungsweise deren Untereinheiten , jetzt auch rekombinant verfügbar, wie z.B. TFIIA, TFIIB, TFIIEα, TFIIEβ und TFIIF (Zawel, L. und Reinberg, D. (1995) Annu Rev. Biochem. 64, 533-561).

Es gibt deshalb heute bereits Transkriptionssysteme, die aus einer Mischung von rekombinanten und natürlichen gereinigten Faktoren bestehen. Für ein Screeningverfahren mit hohem Probendurchsatz, sind solche Transkriptionssysteme bisher jedoch technisch zu aufwendig und zu teuer. In anderen Transkriptionssystemen, z.B. unter Verwendung von Extrakten aus Zellkernen anstelle von rekombinanten oder gereinigten Faktoren, treten dagegen viele Nebenreaktionen auf. In unzureichend oder nicht aufgereinigten Kernextrakten (grobe Extrakte) wirken sich vor allem Nukleinsäuren und DNA-bindene Proteine, beispielsweise Repressoren wie z.B. Histone störend auf die in vitro Transkription aus. Bei den in groben Extrakten enthaltenen Nukleinsäuren stören insbesondere DNA-Sequenzen, die für t-RNAs kodieren. Da die Gene für t-RNAs etwa 100 mal stärker transkribiert werden als die von mRNAs, führen diese t-RNA kodierenden Sequenzen zu einem Überschuß an unspezifischen Transkripten. Die unspezifischen Transkripte müssen dann erst durch aufwendige Reinigungsschritte abgetrennt werden, bevor die spezifischen Transkripte nachgewiesen werden können.

Um die Ergebnisse von in vitro Transkriptionen quantitativ bestimmen zu können, wurden Vektoren entwickelt, bei denen die zu transkribierende DNA-Sequenz keine Guanin-Basen enthält (eine sogenannte G-freie Sequenz bzw. G-freie Kassette) wobei sich an die G-freie Sequenz gegebenfalls ein Sequenzabschnitt anschließt, der sehr viele Guanine enthält. Durch die Verwendung dieser Vektoren ist es möglich, daß die Transkription in Abwesenheit von GTP durchgeführt wird. Dadurch werden nur G-freie Sequenzen, nicht aber andere, G-enthaltende Sequenzen transkribiert. Auf diese Weise werden spezifische Transkripte erhalten, die darüber hinaus eine (nahezu) einheitliche Länge aufweisen. Sawadogo und Roeder haben erstmals die Verwendung eines Vektors für Transkriptionen beschrieben, bei dem eine 400 Nukleotide lange Sequenz unter der Kontrolle des ML-(adenovirus major late) Promotors vorliegt. Mit diesem Vektor werden Transkripte mit einer Länge von etwa 400 Nukleotiden erhalten (Sawadogo, M. und Roeder, R.G. (1985) Proc. Natl. Acad. Sci. USA 82, 4394-4398).
Mit Hilfe dieser Vektoren wurden deutlich weniger unspezifische Transkripte erhalten, weshalb die Verwendung dieser Vektoren in Transkriptionsreaktionen seitdem vielfach beschrieben wurde (Goppelt, A., Stelzer, G., Lottspeich, F., Meisterernst, M. (1996) EMBO J. 15, 3105-3115; Kretzschmar, M., Kaiser, K., Lottspeich, F., Meisterernst, M. (1994) Cell 78, 525-534; Meisterernst, M. Roy, A.L., Lieu, H.M. and Roeder, R.G. (1991) Cell 66, 981-993).
Bisher wurden allerdings ausschließlich Vektoren verwendet, bei denen die G-freie Sequenz eine Länge von 400 Nukleotiden nicht überschreitet.

Um die Ergebnisse der bisher beschriebenen Transkriptionsverfahren quantitativ und qualitativ zu bestimmen, werden die Transkription in Gegenwart von radioaktiv markierten Nukleotiden durchgeführt und die radioaktiv markierten Transkripte nach Phenolisierung und Fällung auf einem Gel aufgetrennt. Auf diese Weise werden nicht nur falsch initiierte oder falsch terminierte Transkripte sowie unspezifisch markierte Nukleinsäuren (z.B. durch das Plasmid verursachte Transkripte oder tRNAs) sondern auch überschüssige Nukleotide von dem spezifischen Transkript abgetrennt. Das Verhältnis der Aktivitäten von überschüssigen radioaktiv markierten Nukleotiden zu radioaktiv markierten Transkripten beträgt in ungünstigen Fällen etwa 10.000: 1, so daß die markierten Transkripte etwa 10.000-fach angereichert werden müssen. Diese Anreicherung des spezifischen Transkripts wird durch die Fällungsschritte und die elektrophoretische Auftrennung erreicht. Diese Anreicherungsschritte sind aber für ein automatisiertes Massenscreening nicht geeignet, weshalb alternative Verfahren entwickelt werden müssen, um markierte Nukleotide in einem Maße zu entfernen, die noch eine quantitative Auswertung der Ergebnisse der Transkription erlauben.

Die Transkription kann auch durch Auftragen der Reaktionslösung auf eine Membran, beispielsweise eine DEAE-Cellulose Membran verfolgt werden. Die radioaktiv markierten Transkripte können dann direkt auf der Membran detektiert werden. Allerdings wurde die Verwendung von Membranen bisher nur für den Nachweis von Transkripten aus in vitro Transkriptionen, die in Gegenwart von gereinigten RNA Polymerasen II (Roeder, R.G. (1974) J. Biol. Chem. 249, 241-248) oder gereinigten basalen Transkriptionsfaktoren (Ohkuma, Y., Sumimoto, H., Horikoshi, M., Roeder, R.G. (1990) Proc. Natl. Acad. Sci. USA 87, 9163-9167) durchgeführt wurden, erfolgreich eingesetzt. Es gibt keinerlei Hinweise darauf, daß Transkripte, die mit Hilfe von angereicherten und gegebenfalls vorgereinigten Extrakten aus Zellkernen erhalten werden, auf diese Weise detektierbar wären.

Die Transkription von Genen für ein Wirkstoffscreening zu nutzen wurde bereits in WO 96/26959 beschrieben. Dort werden die Sequenzen humaner NFATs (hNFAT) und deren mögliche Verwendung in Transkriptionsassays, die wiederum zum möglicherweise automatisierten Massenscreening von Naturstoffen eingesetzt werden sollen, offenbart. Im Gegensatz zu dem im folgenden beschrieben Transkriptionsverfahren, handelt es sich bei diesem Transkriptionsassay um einen reinen Bindungsassay, bei dem keine Transkriptionsreaktion durchgeführt wird.

Ein weiterer Bindungsassay, der zum Screening von Substanzen, die die Bindung von Transkriptionsfaktoren an Nukleinsäuren inhibieren können, verwendet werden kann, wurde in US 5,563,036 beschrieben. Auch bei diesem Assay wird keine Transkription durchgeführt.

Ein weiteres Beispiel für einen Bindungsassay, der ebenfalls zur Auffindung von Substanzen eingesetzt werden soll, die die Bindung von, in diesem Falle einem Tumornekrosefaktor- Rezeptor assozierten Protein (TRADD) an bestimmte DNA-Sequenzen inhibieren können, ist in US 5,563,039 beschrieben.

Eine Aufgabe der vorliegenden Erfindung ist es, ein einfach und reproduzierbar durchzuführendes, universell einsetzbares, insbesondere für ein Massenscreening geeignetes Verfahren zur Analyse der Transkription von Genen, z.B. von zellulären und viralen Genen unter definierten Reaktionsbedingungen bereitzustellen.

Die Erfindung betrifft ein Verfahren zur Identifizier und von pharmakologisch aktiven wirkstoffen umfassend die zellfreie in vitro Transkription eines DNA-Templates, welches eine zu transkribierende DNA-Sequenz, die unter der Kontrolle eines oder mehrerer genregulatorischer Elemente vorliegt, enthält, wobei das Verfahren umfasst :
a) Mischung mit mindestens einem markierten Nukleotid einem Kernextrakt und eines zu testenden und Durchführung der Transkriptionsreaktion.
b) im Anschluß an die Transkription die im Ansatz enthaltenen Proteine abgetrennt und/oder degradiert werden,
c) das markierte Transkript an einen festen Träger gebunden wird,
d) die überschüssigen markierten Nukleotide entfernt werden und
e) die Menge an markiertem Transkript relativ zu einem parallelen Ansatz welcher Abwesenheit des zu bestenden wirkstoffs durch geführt wird, bestimmt wird,

Das für das erfindungsgemäße Verfahren verwendete DNA Template enthält singuläre Schnittstellen für die Restriktionsendonukleasen Pstl, EcoRI, Sacl, Kpnl, SacII, BamHI, Swal und SmaI, wobei fünf Bindungsstellen für das Hefe Gal 4 Protein und die "TATA"-Box des humanen T-Zellrezeptors Vβ 8.1 zwischen den Restriktionsschnittstellen für SacII und BamHI, die INR (Initiator)-Region des ML Promotors (adenovirus major late promotor) zwischen den Restriktionsschittstellen für BamHI und Swal und eine G-freie Sequenz mit einer Länge von etwa 800 Nukleotiden (Basenpaaren) zwischen den Restriktionsschnittstellen Swal und Smal in das pUC19 Plasmid kloniert sind.

Das Verfahren beinhaltet die eigentliche Transkriptionsreaktion (a), die Abtrennung des spezifischen Transkripts (b, c, d) und die Detektion des spezifischen Transkripts (e).

Das Verfahren beinhaltet besondere Ausführungsformen für die Abtrennung des spezifschen Transkripts, wobei die genannte Reihenfolge von b, c und d nur eine Möglichkeit ist. Die Reihenfolge der Abtrennung des spezifischen Transkripts kann gegebenfalls auch c, d, b oder d, b, c sein.

Ein besonderes Merkmal des Verfahrens ist, daß alle Verfahrenschritte, also die eigentliche Transkriptionsreaktion (Transkription) sowie die Abtrennung und Detektion des spezifischen Transkripts automatisierbar sind, wobei eine einfache und zuverlässige Bestimmung der Menge des unter den jeweiligen Reaktionsbedingungen erhaltenen spezifischen Transkripts und damit der Transkriptionsrate möglich ist.

Die Transkriptionsrate gibt an, wie oft ein bestimmtes Gen pro Zeiteinheit transkribiert wird bzw. im beschriebenen Transkriptionsverfahren, wie oft die zu transkribierende DNA-Sequenz pro Zeiteinheit transkribiert wird. Um die Transkriptionsrate zu bestimmen, wird die Menge an radioaktiv markiertem Transkript, die nach einer definierten Zeiteinheit erhalten wird, bestimmt.

Das Verfahren beinhaltet, daß die Transkription in Gegenwart von Aktivatoren und/oder Inhibitoren durchgeführt wird, d.h. in Gegenwart von Komponenten, die die Transkription positiv oder negativ beinflussen. Erfindungsgemäß wird ein transkriptionsfähiger Extrakt aus Zellkernen für die basale Transkription eingesetzt . Dieses basale Transkriptionssystem kann durch Aktivatoren und/oder Inhibitoren ergänzt werden. Im Vergleich zur basalen Transkription führt eine Inhibierung der Transkription zu einer verminderten Transkriptionsrate und damit zu einer geringeren Menge an spezifischem Transkript/Zeiteinheit, während eine Aktivierung der Transkription zu einer erhöhten Transkriptionsrate und damit zu einer größeren Menge an spezifischem Transkript/Zeiteinheit führt.

Die Transkription von Genen läßt sich in mehrere Schritte einteilen - die Bildung des Pre-Initiations-Komplexes (PIC), Aktivierung des PIC, Initiation, "Promotor Clearance", Elongation und Termination. Für die Initiation der Transkription bei Eukaryoten sind RNA-Polymerasen (für die Transkription von proteinkodierenden Genen RNA Polymerase II) und DNA-bindende Proteine, die die spezifische Wechselwirkung der RNA Polymerase II mit der DNA ermöglichen, nötig. Diese DNA-bindenen Proteine bezeichnet man als Transkriptionsfaktoren, wobei die generellen Transkriptionsfaktoren im wesentlichen an der Wechselwirkung mit dem Promotor beteiligt sind, während die spezifischen Transkriptionsfaktoren die Wirkung genregulatorischer Elemente, die stromabwärts oder stromaufwärts des Promotors lokalisiert sind, vermitteln.

An der Transkription eukaryotischer Gene sind die generellen Transkriptionsfaktoren TFIIA, TFIIB, TFIID, TFIIE, TFIIF und TFIIH beteiligt. Eine transkriptionsfähige Proteinfraktion, die für eine geringfügige, basale Aktivität von Genen verantwortlich ist, enthält je nach Promotor alle bzw. die meisten dieser generellen Transkriptionsfaktoren und RNA Polymerase II (RNA Pol II). Eine basale Aktivität des ML-Promotors wird z.B. durch TBP (TATA-bindende Untereinheit von TFIID), TFIIB, TFIIE, TFIIF, TFIIH und RNA Pol II erreicht. Proteinfraktionen, die eine solche basale Aktivität hervorrufen werden als basale Transkriptionssysteme bezeichnet. Dieser Begriff wird im Sinne der Erfindung auch für einen angereicherten und gebenfalls aufgereinigten Extrakt aus Zellkernen verwendet. Eine Transkription, die mit Hilfe eines basalen Transkriptionssystems durchgeführt wird, wird als basale Transkription bezeichnet. Um eine zellfreie Transkription in vitro durchzuführen, wird mindestens ein basales Transkriptionssystem, Nukleotide und ein zu transkribierendes DNA-Template benötigt.

Für die aktivierte Transkription werden zusätzlich zu den generellen Transkriptionsfaktoren bzw. zu einem basalen Transkriptionssystem spezifische Transkriptions- und Kofaktoren (accessorische Proteine) benötigt (Kaiser, K., Stelzer, G. und Meisterernst, M. (1995) EMBO J. 14, 3520-3527). Spezifische Transkriptionsfaktoren sind in der Lage, die nur geringe basale Transkription bestimmter Gene um ein vielfaches zu verstärken und die Häufigkeit der Transkriptionsinitiation zu steuern. DNA-bindende Proteine sind auf diese Weise maßgeblich dafür verantwortlich, wie oft ein Gen transkribiert wird (Transkriptionsrate). An diesem Regulationsprozess sind auch andere Proteine, die nicht direkt an die DNA binden, sondern über Protein-Protein Wechselwirkungen die Aktivitäten von Transkriptionsfaktoren oder RNA-Polymerasen II beinflussen, wie z.B. Kofaktoren, beteiligt.

Das Verfahren beinhaltet, daß für die basale Transkription ein angereicherter Extrakt aus Zellkernen (Zellkernextrakt bzw. Kernextrakt) eingesetzt wird. Im Bezug auf diesem Parameter ist das Verfahren universell einsetzbar; das gilt sowohl für die verwendete Zelle, als auch für die verwendete eukaryotische Spezies. Beispielsweise können z.B. angereicherte Kernextrakte aus von menschlichen oder tierischen Zellen abgeleiteten Zellinien gewonnen werden. Insbesondere sind Zellen geeignet, die in Fermentern im großen Maßstab kultiviert und vermehrt werden können, wie z.B. HeLa Zellen. Weiterhin können Extrakte aus den Zellkernen ausgewählter Zelltypen, insbesondere von solchen, die sich z.B. durch ihre zelltypspezifische, zellzyklusspezifische, entwicklungsspezifische, differenzierungsspezifische oder erkrankungsspezifische Ausstattung mit Transkriptions- und/oder Kofaktoren auszeichnen, verwendet werden. Insbesondere können Zelltypen verwendet werden, denen eine zentrale Rolle bei der Entstehung von Krankheiten zukommt, wie z.B. Zellen des Immunsystems (z.B. B- und T-Zellen).

Ein besonderer Vorteil des Verfahrens ist, daß auch Kernextrakte aus Geweben oder Tumorzellen isoliert und verwendet werden können. Dies ist besonders vorteilhaft in den Fällen, in denen keine geeignete Zelline verfügbar ist. Insbesondere können Kernextrakte aus leicht zugänglichen Geweben, wie aus tierischen oder menschlichen Nabelschnüren, tierischen oder menschlichen Transplantations-Abfallprodukten, tierischem oder menschlichem Biopsiematerial oder tierischem oder menschlichem Tumorgewebe (z.B. operativ entferntes Gewebe), tierischer oder menschlicher Placenta isoliert und für das Verfahren eingesetzt werden.

Das Verfahren beinhaltet, daß für die Anreicherung von Kernproteinen aus den Zellkernen von frischen oder tiefgefrorenen Zellen oder aus frischen oder tiefgefrorenen Zellkernen, angereicherte Kernextrakte nach bekannten Verfahren hergestellt werden, beispielsweise nach einer Methode, die von Dignam et al. beschrieben wurde (Dignam, J.D., Martin, P.L., Shastry, B.S., Roeder, R.G. (1983) Methods in Enzymology 101, 582-598; Dignam, J.D., Lebovitz, R.M., Roeder, R.G. (1983) Nucleic Acid Res. 11, 1475-1489). Eine besondere Ausführungsform des Verfahrens ist, daß für die Herstellung eines angereicherten Kernextrakts Verfahren benutzt werden, die eine Homogenisation der Zellkerne mit anschließender Dialyse des Homogenats beinhalten.

Eine wichtige Ausführungsform des Verfahrens beinhaltet, daß der angereicherte Extrakt aus Zellkernen durch einen oder mehrere Reinigungsschritte, insbesondere einfache Reinigungsschritte in dem Maße aufgereinigt wird, daß er transkriptionsfähig ist, d.h. das bei Durchführung des Verfahrens ein spezifisches Transkript erhalten wird. Beispielsweise kann der Extrakt chromatographisch aufgereinigt werden. Die Reinigung kann z.B. über kernproteinbindende Materialien wie Phosphocellulose, DEAE-Cellulose oder auch Heparin-Sepharose erfolgen. Alternativ können für die Reinigung Kationen- und/oder Anionenaustauschersäulen oder spezifische Affinitätssäulen, z.B. solche, bei denen an das Säulenmaterial Antikörper oder Oligonukleotide gebunden sind, eingesetzt werden.

Eine besondere Ausführungsform des Verfahrens beinhaltet, daß ein angereicherter Kernextrakt über eine Phosphocellulosesäule, insbesondere eine P11®-Säule (P11®-System, Whatman, Maidstone, England) aufgereinigt wird. Eine weitere besondere Ausführungsform des Verfahrens beinhaltet, daß der angereicherte Kernextrakt nur über einen einzigen Schritt aufgereinigt wird, beispielsweise über eine einzige P11®-Säule, oder eine einzige Säule mit Säulenmaterial, das DEAE-Cellulose oder Heparin-Sepharose enthält.

Bei einer speziellen Ausführungsform der P11®-Reinigung wird der Kernextrakt zuerst in Gegenwart eines Puffers, der neben anderen Bestandteilen, 0.05 bis 0.15 M, vorzugsweise 0,1 M KCI enthält, an die Phosphocellulose gebunden. Durch das Waschen der beladenen Säule mit geeigneten Puffern, vorzugsweise mit einem Puffer, der 0,05 bis 0,15 M KCI, vorzugsweise 0,1 M KCI enthält, werden unspezifische und störende Komponenten von der Säule gewaschen. Die transkriptionsfähigen Komponenten werden vorzugsweise in zwei Fraktionen von der Säule eluiert, wobei zuerst ein Puffer der beispielsweise 0,4 bis 0,6 M KCI, vorzugsweise 0,5 M KCI enthält, und im Anschluß ein Puffer, der beispielsweise 0,7 bis 1 M KCI, vorzugsweise 0,85 M KCI enthält, verwendet wird.

Eine spezielle Ausführungsform des Verfahrens ist, daß die Transkription mit Hilfe eines angereicherten, gegebenfalls aufgereinigten Kernextrakts in Gegenwart von exogener RNA Polymerase II durchgeführt wird. Als RNA-Polymerase kann vorzugsweise eukaryotische RNA-Polymerase vom Typ II (RNA-Polymerase II), insbesondere tierische oder menschliche RNA-Polymerase II verwendet werden.

Eine weitere Ausführungsform des Verfahrens beinhaltet, daß ein angereicherter und gegebenenfalls aufgereinigter Kernextrakt durch Zusatz von Proteinen, beispielsweise von Transkriptionsfaktoren und/oder Kofaktoren (accessorischen Proteinen) ergänzt beziehungsweise ganz oder teilweise ersetzt werden kann. Diese Proteine können beispielsweise aus den Kernen von Zellen aufgereinigt oder rekombinant hergestellt werden.

Eine spezielle Ausführungsform des Verfahrens ist, daß der Kernextrakt nur durch einen Transkriptions- und/oder Kofaktor ergänzt wird. Im anderen Extrem beinhaltet das Verfahren, daß eine transkriptionsfähige Protein-Fraktion (basales Transkriptionssystem) vollständig aus aufgereinigten oder rekombinant hergestellten Transkriptions- und/oder Kofaktoren sowie RNA-Polymerasen zusammengestellt wird.

Als Transkriptionsfaktoren können beispielsweise generelle und/oder spezifische Transkriptionsfaktoren bzw. Teile derselben, gegebenfalls als Fusionsproteine, eingesetzt werden.

Als generelle Transkriptionsfaktoren können beispielsweise TFIIA, TFIIB, TFIID, TFIIE, TFIIF, TFIIH, TFIIJ und TBP (TATA-binding protein) eingesetzt werden.

Als spezifische Transkriptionsfaktoren können beispielsweise NFκB, AP1, NFAT, GATA3, TCF/Lef, CBF, Tat, Mitglieder der fos/jun Familie, der Oct Familie (Oct-1, Oct-2) und damit wechselwirkende Faktoren wie z.B. Bobl, OCA-B oder OBF, der Ets Familie, Aktivatoren der Familie der ATF/CREB Proteine, nukleäre Rezeptoren wie z.B. PPARα oder die entsprechend zelltyp-spezifischen Isoformen von Transkriptionsfaktoren verwendet werden (Kel, O.V., Romaschenko, A.G., Kel, A.E., Wingender, E., Kolachenov, N.A., (1995) Nucl. Acids. Res. 20, 3-16).

Weitere Beispiele für spezifische Transkriptionsfaktoren sind:
1. Protoonkogene, beispielsweise jun, fos, ets, myc, bc-Isoformen und erb
2. Hormonrezeptoren, beispielsweise (erb), Glukokortikoid-, Estrogen-, Retinolsäure-, Vitamin D-Rezeptoren oder
3. Tumorsupressoren, beispielsweise p53, NF1, WT1, RB
4. virale Pathogene, beispielsweise Proteine des Herpes Simplex-Virus, wie z.B. VP16 oder ICP4, des Papillomavirus, wie z.B. E1, E2, E6 oder E7, des Adenovirus, wie z.B. E1A oder E2A, des Cytomegali-Virus, wie z.B. IE86, des Hepatitis B-Virus, wie z.B. pX, des HIV-Virus, wie z.B. Tat oder Rev
5. zelltyp- und/oder gewebespezifische Faktoren, wie beispielsweise myogene Faktoren, Pit-1, Oct-2, Pu-1, OCA-B oder HNFs oder T-Zell spezifische Faktoren wie Ets-1, GATA3, TCF/Lef, CBF,
6. STAT-Proteine (Signal transducers and activators of transcription) beispielsweise Zytokin-aktivierte Transkriptionsfaktoren, wie z.B. IL-1 Stat, IL-2 Stat, IL-3 Stat, IL-4, IL-5 Stat, IL-6 Stat, IL-7 Stat, IL-8 Stat, IL-9 Stat, IL-10 Stat, IL-11 Stat, IL-12 Stat ("Stat" bedeutet Protein, das die Wirkung vermittelt) oder
7. Proteine, die an second messenger Transduktionskaskaden beteiligt sind, beispielsweise CREB oder abl,
8. nukleäre Rezeptoren, beispielsweise "second messenger"-Rezeptoren (beispielsweise cAMP- oder IP₃-Rezeptoren, Ca²⁺-abhängige Rezeptoren), Retinolsäure-Rezeptoren, Glukokortikoidrezeptoren oder Steroidrezeptoren,
9. genspezifische Aktivatoren oder Inhibitoren, beispielsweise spezifische Aktivatoren des IL-2-Gens, wie NFκB, AP1 oder NFAT,
10. entwicklungsspezifisch, zellzyklusspezifisch und differenzierungsabhängig exprimierte Transkriptionsfaktoren.

Kofaktoren sind beispielsweise über Protein-Protein-Wechselwirkungen und/oder Protein-DNA-Wechselwirkungen direkt oder indirekt an der Transkription beteiligt. Einige Kofaktoren sind bereits in einem basalen Transkriptionssystem vorhanden, andere erst im aktivierten Transkriptionssystem. Kofaktoren können die Transkriptionsrate positiv oder negativ beeinflussen. Als Kofaktoren können beispielsweise
- TBP-assoziierte Faktoren (TAFs), z.B. TAF_{II}30, TAF_{II}40, TAF_{II}55, TAF_{II}60, TAF_{II}110, TAF_{II}150 TAF_{II}250 (Verrijzer, C.P. und Tjian, R. (1996) Trends Biochem. Sci. 21, 338-342; TAFs bilden zusammen mit TBP den TFIID-Komplex, wobei die Zusammensetzung der TAFs im TFIID erheblich variieren kann);
- Mediatoren, d.h. Kofaktoren, die mit der RNA Polymerase II assoziiert sind, wie beispielsweise CTD (carboxyterminal domain)-interagierende Proteine und/oder Repressoren und/oder Aktivatoren der RNA Polymerase II, insbesondere RAP 30, RAP 74, RAP 38, SR7 (Suppressor der RNA Polymerase B, SRB), Zykline oder Kinasen (z.B. CKII);
- Generelle Kofaktoren;
- Kofaktoren, die in der USA (upstream stimulatory activity)-Fraktion enthalten sind (Kaiser, K. und Meisterernst, M. (1996) Trends Biochem. Sci., 342-345);
- Positive Kofaktoren, beispielsweise PC1, PC2, PC4 (p15), PC5, PC6, Dr2 (D Repressor 2)/PC3, ACF(Aktivierender Kofaktor), CofA (Kofaktor A), HMG-Proteine (chromatin-assoziated high mobility group proteins);
- Negative Kofaktoren, beispielsweise NC1, NC2 und/oder
- Spezifische Kofaktoren
eingesetzt werden.

Das Verfahren beinhaltet, daß für die Transkription ein DNA-Template eingesetzt wird, das ein oder mehrere genregulatorische Elemente und eine zu transkribierende DNA-Sequenz enthält.

Ein Gegenstand der Erfindung ist ein DNA-Template, das in dem beschriebenen Verfahren zur zellfreien in vitro Transkription eingesetzt werden kann. Das DNA-Template enthält ein oder mehrere genregulatorische Elemente und eine zu transkribierende DNA-Sequenz. Darüber hinaus enthält das erfindungsgemäße DNA-Template singuläre Schnittstellen für die Restriktionsendonukleasen PstI, EcoRI, Sacl, Kpnl, Sacll, BamHI, Swal, Smal, fünf Bindungsstellen für das Hefe Gal 4 Protein, die "TATA"-Box des humanen T-Zellrezeptors Vβ 8.1 zwischen den Restriktionsschnittstellen für SacII und BamHI, die INR (Initiator)-Region des ML-Promotors (adenovirus major late promotor) zwischen den Restriktionsschnittstellen BamHI und Swal und eine G-freie Sequenz mit einer Länge von etwa 800 Nukleotiden (Basenpaaren) in dem pUC19 Plasmid.

Ein genregulatorisches Element kann ein bekanntes oder zu untersuchendes genregulatorisches Element oder ein Konstrukt aus einem oder mehreren bekannten und einem oder mehreren zu untersuchenden genregulatorischen Elementen sein.

Ein genregulatorisches Element kann beliebige an der Genregulation beteiligte DNA-Sequenzen oder Abschnitte davon enthalten. Im Bezug auf das genreguatorische Element ist das DNA-Template bzw. das Verfahren universell, wobei das genregulatorische Element vorzugsweise aus einem eukaryotischen Gen stammt beziehungsweise diesem entspricht. Das genregulatorische Element kann ein zelluläres oder virales genregulatorisches Elemente oder ein synthetisches genregulatorisches Element sein. Vorzugsweise enthält ein genregulatorisches Element unter anderem DNA-Sequenzen, die Bindungsstellen für DNA-bindende Proteine (Protein-bindende DNA-Sequenzen, beispielsweise Bindungsstellen für Transkriptionsfaktoren oder Fusionsproteine) darstellen. Ein genregulatorisches Element kann einen Promotor (Promotorsequenz) und/oder einen oder mehrere Enhancer (Enhancersequenz) und/oder einen oder mehrere Silencer (Silencersequenz) enthalten. Vorzugsweise kann das genregulatorische Element natürlich und/oder künstlich angeordnete Promotor-, Enhancer- und/oder Silencersequenzen oder Teile davon enthalten.

Ein Promotor kann eine "TATA" -Box und/oder eine Initiator-Region (INR) (Transkriptionsstartpunkt) enthalten. Der Promotor kann eine "GC"-Box und/oder eine "GAAT"-Box enthalten.

Erfindungsgemäß ist das genregulatorische Element des DNA Templats ein Modellpromotor.

Ein Modellpromotor enthält einen Promotor und zusätzliche proteinbindende DNA-Sequenzen und gegebenfalls weiter genreguatorische Elemente. Vorzugsweise enthält der Modellpromotor eine "TATA"-Box und eine Initiatorregion. Gemäß vorliegenden Erfindung enthält der Modellpromotor die "TATA"-Box des humanen T-Zellrezeptors Vβ8.1 und die Initiator-Region des ML-Promotors. Diese beiden basalen Promotorelemente ermöglichen eine basale Transkription in vitro. Darüber hinaus hat dieser spezielle Modellpromotor 5 Bindungsstellen für das Hefe Gal4 Protein. Der Modellpromotor kann beliebig, z.B. mit Hilfe von molekularbiologischen Methoden verändert werden, beispielsweise indem der Modellpromotor durch, z.B. ein zu untersuchendes genregulatorisches Element ergänzt wird und/oder dadurch, daß einzelne Bereiche des Modellpromotors durch weitere genregulatorische Elemente, z.B. ein zu untersuchendes genregulatorisches Element ersetzt wird.

Um eine Manipulation des Modellpromotors zu ermöglichen, enthält dieser erfindungsgemäß singuläre Schnittstellen für Restriktionsendonukleasen, wobei mindestens eine singuläre Schnittstelle für eine Restriktionsendonuklease zwischen der "TATA"-Box und den Gal4-Bindungsstellen lokalisiert ist.

In den Modellpromotor können zusätzlich zu den bereits vorhandenen proteinbindenden (z.B. zusätzlich zu den Gal4 Sequenzen), weitere proteinbindende Sequenzen integriert werden. Das ist insbesondere dann interessant, wenn beispielsweise zusätzlich zu einem bereits untersuchten Transkriptionssystem weitere zu untersuchende, z.B. spezifische Transkriptionsfaktoren zugegeben werden.

Das Verfahren beinhaltet, daß in Kombination mit dem Modellpromotor auch Fusionsproteine als Aktivatoren und/oder Inhibitoren der Transkription eingesetzt werden können. Solche Fusionsproteine können beispielsweise aus einer DNA-bindenden Domäne, wie beispielsweise der DNA-bindenden Domäne des Hefe Gal4 Proteins und einer spezifischen Aktivierungsdomäne, wie beispielsweise der Aktivierungsdomäne des HSV Aktivators VP16 bestehen. Mit Hilfe von Fusionsproteinen können definierte Aktivatoren und/oder Inhibitoren beziehungsweise Teile derselben, z.B. deren Aktivierungs- beziehungsweise Inhibierungsdomänen im Bezug auf ein zu untersuchendes genregulatorisches Element hintergrundfrei analysiert werden. Beispielsweise ist dies möglich, wenn die DNA-bindende Domäne von einem DNA-bindenden Protein stammt, das in dem verwendeten Transkriptionssystem (z.B. dem angereicherten Kernextrakt) nicht enthalten ist. Beispielsweise kann die Aktivator(-domäne) eines Transkriptionsfaktors, die als Fusionsprotein mit einer Hefe Gal4-Bindungsdomäne vorliegt, hintergrundfrei analysiert werden, wenn angereicherte Kernextrakte aus Säugetierzellen in dem Verfahren eingesetzt werden, da Kernextrakte aus Säugetierzellen kein Gal4 enthalten.

Als genregulatorische Elemente und zu untersuchende genregulatorische Elemente können definierte menschliche und/oder tierische und/oder virale genregulatorische Elemente, insbesondere die genregulatorischen Elemente von pathologisch interessanten Genen verwendet werden. Beispiele hierfür sind die genregulatorischen Elemente der Gene von Adhäsionsmolekülen, Wachstumsfaktoren, Phosphodiesterasen, Phosphatasen, Kinasen, ATPasen, Membranrezeptoren, second messenger Rezeptoren, Hormonrezeptoren, z.B. Steroidrezeptoren, Metalloproteasen, Immunophilinen, NO-Synthasen, 5-Lipoxygenasen oder immunologische Targets, wie die genregulatorischen Elemente von Zytokinen, z.B. von Interleukinen, wie die Promotoren von T- oder B-zellspezifisch exprimierten Genen, z.B. vom CD4 Rezeptor, TCR oder BCR (T- oder B-Zellrezeptoren), wie die Promotoren von lymphoidspezifischen Genen, z.B. vom TNF (Tumornekrosefaktor) oder wie die genregulatorischen Elemente T-zellspezifischer Retroviren, z.B. von HTLV-1 oder HIV-1.

Ein besonderer Vorteil des Verfahrens bzw. des Modellpromotors ist, daß als genregulatorische Elemente sowohl solche Promotoren verwendet werden können, die eine "TATA"-Box enthalten, wie z.B. der Promotor des Gens, das für Interleukin-2 (IL-2) kodiert als auch Promotoren, die keine "TATA"-Box enthalten, wie z.B. der Promotor des Gens, das für die β-Kette des T-Zellrezeptors kodiert: Beispielsweise können Promotoren, die keine "TATA"-Box enthalten in den Modellpromotor bzw. das universelle Reporterplasmid pGS100 integriert und für die Transkription eingesetzt werden.

Die zu transkribierende DNA-Sequenz ist dadurch gekennzeichnet, daß eine oder mehrere Nukleobasen in dieser Sequenz nicht enthalten sind. Vorzugsweise enthält die zu transkribierende DNA-Sequenz wahlweise entweder kein Guanin oder kein Cytosin oder kein Thymin, d.h. die Sequenz ist G-frei, C-frei oder T-frei. Weiterhin kann die Sequenz auch mehrere Nukleobasen, wie beispielsweise Guanin und Thymin oder Guanin und Cytosin oder Cytosin und Thymin nicht enthalten.

Insbesondere werden G-freie, T-freie oder C-freie Sequenzen verwendet, bei denen die G-freie, T-freie oder C-freie Sequenz eine Länge von etwa 800 Nukleotiden aufweist.

Das DNA-Template kann eine lineare oder zirkuläre DNA-Sequenz sein, beispielsweise kann das DNA-Template eine mit der Polymerase Kettenreaktion hergestellte lineare Sequenz oder ein Plasmid sein.

Eine Ausführungsform ist, daß das DNA-Template ein Plasmid ist und aus einem ganzen oder einem Teil eines Vektors, einem Modellpromotor und einer zu transkribierenden DNA-Sequenz, die beispielsweise G-frei, T-frei oder C-frei ist, aufgebaut ist.

Gegenstand der Erfindung ist ein universell einsetzbares Reporterplasmid (Universelles Reporterplasmid). Das universelle Reporterplasmid enthält singuläre Schnittstellen für die Restriktionsengonukleasen PstI, EcoRI, SacI, KpnI, SacII, BamHI, Swal, einen Teil.des pUC19 Plasmids, fünf Bindungstellen für das Hefe Gal4 Protein, die "TAIA"-Box des humanen T-Zellrezeptors Vβ 8.1 zwischen den Restriktionsschnittstellen für SacII und BamHI, die INR (Initiator)-Region des ML-Promotors (adenovirus major late promoter) zwischen den Restriktionsschnittstellen BamHI und Swal und eine G-freie Sequenz mit einer Länge von etwa 800 Nukleotiden (Basenpaaren)). Der Modellpromotor in dem universellen Reporterplasmid ist ein synthetischer Promotor, der 5 Hefe Gal4 Bindungsstellen, die singulären Schnittstellen für PstI, EcoRI, Sacl, Kpnl, SacII, BamHI und SwaI, die TATA-Box des humanen T-Zellrezeptors Vβ 8.1 und die INR des ML Promotors enthält. Beliebige zu untersuchende genregulatorische Elemente können in diese Promotorregion integriert werden. Es können Teile des Modellpromotors entfernt und durch zu untersuchende genregulatorische Elemente ersetzt werden.

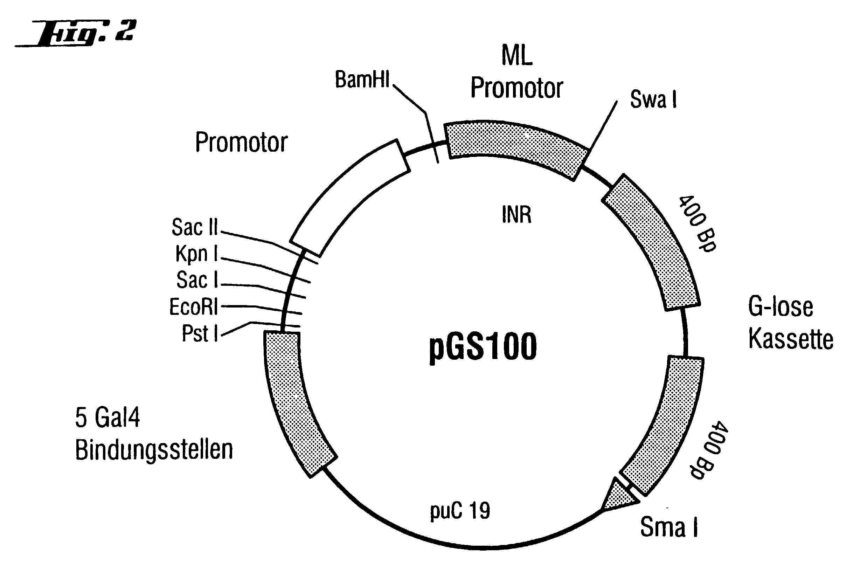

Eine Ausführungsform des universellen Reporterplasmids wird als pGS100 bezeichnet (Fig. 2).

Eine Ausführungsform des universellen Reporterplasmids pGS100 ist dadurch gekennzeichnet, daß sich der synthetische Promotor im Sequenzbereich zwischen den Nukleotidpositionen 2168 und 2337 befindet. Der Initiatorbereich des adenoviralen "major late" (ML) Promotors befindet sich an den Nukleotidpositionen 2322 bis 2337 und der Bereich mit TATA-Box des humanen T-Zellrezeptor Vβ 8.1 Promotors an den Nukleotidpositionen 2289 bis 2316. Die fünf Bindungsstellen für das Hefe Gal4 Protein befinden sich zwischen den Nukleotidpositionen 2168 bis 2260.

Eine weitere Ausführungsform des universellen Reporterplasmids pGS100 ist durch die Nukleotidsequenz (SEQ ID NO. 1) in Tabelle 1 gegeben.

Eine weitere Ausführungsform des universellen Reporterplasmids pGS100 wurde bei der DSMZ-Deutsche Sammlung von Mikroorganismen und Zellkulturen GmbH, Mascheroder Weg 1b, D-38124 Braunschweig unter der DSM-Nummer 11450 gemäß den Bestimmungen des Budapester Vertrages über die internationale Anerkennung der Hinterlegung von Mikroorganismen für die Zwecke von Patentverfahren hinterlegt.

Das Verfahren beinhaltet, daß Proteine, die die Detektion des spezifischen Transkripts direkt oder indirekt in der Weise stören, daß kein eindeutiger Nachweis der spezifischen Transkripte mehr möglich ist, im Anschluß an die Transkription abgetrennt und/oder degradiert werden. Die Abtrennung oder Degradierung beinhaltet insbesondere einfach durchzuführende Verfahrensschritte, wie beispielsweise solche, bei denen die Reaktion durch einen chemischen und/oder mechanischen und/oder enzymatischen Verfahrensschritt gestoppt und die Transkripte gegebenfalls gleichzeitig von störenden Proteinen in der Weise befreit werden, daß im Anschluß an diesen Verfahrensschritt überschüssige markierte Nukleotide, beispielsweise durch Waschschritte von den spezifischen Transkripten abtrennt werden können.

Diese Ausführungsform des Verfahrens beinhaltet, daß beispielsweise im Anschluß an die Transkriptionsreaktion die Proteine mit Hilfe von Proteasen degradiert werden können. Als Proteasen können beispielsweise Zinkproteasen, Serinproteasen, Thiolproteasen und Carboxyproteasen verwendet werden. Insbesondere können Proteinase K, Trypsin, Chymotrypsin, Carboxypeptidase A, Papain und Pepsin eingesetzt werden. Eine besondere Ausführungsform des Verfahrens ist, daß im Anschluß an die Transkription ein Proteinase K Verdau durchgeführt wird.

Um das Ausmaß der Transkription, d.h. die Transkriptionsrate bestimmen zu können, muß die Menge an spezifischemTranskript bestimmt werden.

Das Verfahren beinhaltet, daß die Transkription in Gegenwart von markierten Nukleotiden, durchgeführt wird oder daß das spezifische Transkript markiert wird. Das Transkript kann beispielsweise nichtradioaktiv markiert werden, wenn für die Transkription entsprechende, nichtradioaktiv markierte Nukleotide eingesetzt werden. Als Markierungsgruppen für Nukleotide können z.B. fluoreszierende Gruppen, wie Dansyl-(=N-Dimethyl-1-aminonaphthyl-5-sulfonyl-), Fluorescein- oder Coumarin-Derivate oder chemilumineszierende Gruppen, wie Acridin-Derivate verwendet werden. Die genannten Markierungsgruppen gestatten einen direkten Nachweis des spezifischen Transkripts. Darüber hinaus können auch Markierungsgruppen verwendet werden, die für einen indirekten Nachweis des Transkripts geeignet sind. Beispiele hierfür sind Digoxygenin, welches mit spezifischen Anti-Digoxigenin Antikörpern, z.B. im ELISA, nachweisbar ist, Biotin, welches über das Biotin/Avidin-System nachweisbar ist und Linker-Arme, die funktionelle Gruppen enthalten, die eine nachträgliche Derivatisierung mit einer nachweisbaren Reporter-Gruppe gestatten. Ein Beispiel für die letzgenannte Möglichkeit ist beispielsweise ein Aminoalkyl-Linker, der im Anschluß an die Transkription mit einem Acridinium-Aktivester zur Chemilumineszenz-Probe umgesetzt und nachgewiesen werden kann.

Eine besondere Ausführungsform des Verfahrens ist, daß die Transkription in Gegenwart von radioaktiv markierten Nukleotiden durchgeführt wird. Die Nukleotide können beispielsweise radioaktiv markiert sein mit Phosphor (³²P oder ³³P), Schwefel (³⁵S) oder Tritium (³H).

Eine besondere Ausführungsform des Verfahrens ist, daß die spezifischen Transkripte durch Bindung an eine feste Phase (fester Träger) aus dem Reaktionsgemisch abgetrennt werden, z.B. durch Bindung an eine Mikrotiterplatte oder durch Bindung des spezifischen Transkripts an spezielle Filter, Membranen oder andere Festphasen, insbesondere durch Bindung an eine geladene Membran oder einen geladenen Filter, vorzugsweise aus Nylon oder Nitrocellulose, besonders bevorzugt durch Bindung an eine Membran, die geladene Gruppen, wie z.B. Diethylaminoethylgruppen enthält, wie z.B. eine DEAE-Cellulose Membran. Diese Ausführungsform beinhaltet, daß die an den festen Träger gebundenen spezifischen Transkripte durch Waschschritte von überschüssigen markierten Nukleotiden soweit befreit werden können, daß eine eindeutige Detektion der spezifischen Transkripte möglich ist.

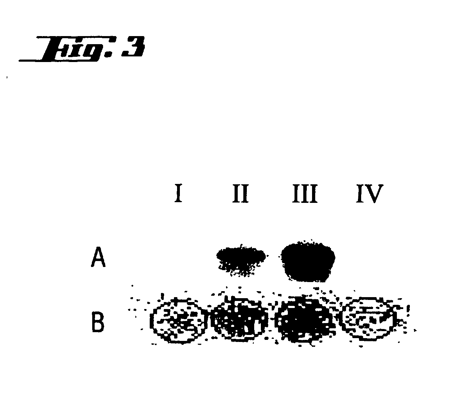

Überraschenderweise werden mit diesem Verfahren im Vergleich zu der herkömmlichen Abtrennung und Detektion, bestehend aus Phenolisierung, Fällung und anschließende Auftrennung der Transkripte auf einem denaturierenden Gel, ebenso eindeutig nachweisbare spezifische Signale erhalten (vergl. Beispiel 7 und Fig. 3).

Das Verfahren ist dazu geeignet, spezifische Signale, ausgelöst z.B. durch die spezifische Transkription in Gegenwart z.B. eines Aktivators (aktivierte Transkription) oder eines Inhibitors (inhibierte Transkription) zu erzeugen, die mindestens um den Faktor 7, vorzugsweise um den Faktor 8, in besonderen Fällen sogar um den Faktor 9 oder 10 von der basalen Signalstärke, ausgelöst durch die basale Transkription z.B. ohne die Gegenwart des Aktivators oder Inhibitors, abweichen (vergl. Beispiel 7 und Fig. 3).

Eine wichtige Ausführungsform des Verfahrens ist, daß es zum Screening von pharmakologisch aktiven Wirkstoffen eingesetzt werden kann. Um potentielle pharmakologische Wirkstoffe im Bezug auf ihre Aktivität (z.B. aktivierende bzw. inhibierende Eigenschaft) zu testen, wird die Transkription in Gegenwart des zu testenden Wirkstoffes durchgeführt. Gegebenfalls können einzelne Komponenten, z.B. der angereicherte Kernextrakt mit dem zu testenden Wirkstoff vorinkubiert werden. Darüber hinaus kann die Spezifität des zu testenden Wirkstoffes charakterisiert werden, indem die Transkription in Gegenwart des zu testenden Wirkstoffes in mehreren Transkriptionsansätzen parallel durchgeführt wird, wobei jeder Ansatz unterschiedliche Komponenten enthält und dann vergleichend die Transkriptionsrate bestimmt wird.

Zu testende Wirkstoffe können beispielsweise Naturstoffe und/oder Substanzen aus chemischen und kombinatorischen Substanzbibliotheken sein. Naturstoffe können beispielsweise aus Pflanzen, Tieren, Sekreten von Pflanzen oder Tieren und insbesondere aus Mikroorganismen, wie z.B. aus Pilzen, Hefen, Bakterien oder Algen isoliert werden.

Ein weiteres Merkmal des erfindungsgemäßen Verfahrens ist, daß die Transkription in Anwesenheit eines zu testenden Wirkstoffs, Transkriptionsfaktors, Kofaktors oder zelltypspezifischen Kernextrakts und ein paralleler Ansatz in Abwesenheit des zu testenden Wirkstoffs. Transkriotionsfaktors, Kofaktors oder zelltypspezifischen Kernextrakts ansonsten aber unter gleichen Bedingungen durchgeführt wird und aus der Differenz der Mengen an markiertem Transkript die Aktivität (z.B. inhibierend, aktivierend) bzw. der Effekt des zu testenden Wirkstoffs, Transkriptionsfaktors, Kofaktors oder zelltypspezifischen Kernextrakts im Bezug auf ein zu untersuchendes genregulatorisches Element (bzw. Gen) und/oder einen Transkriptions- und/oder Kofaktoren ermittelt wird.

Das Verfahren beinhaltet, daß der Effekt eines zu untersuchenden, an der Genregulation beteiligten Proteins mit Hilfe von parallel unter gleichen Bedingungen durchgeführten Transkriptionen ermittelt wird, wobei das zu untersuchende Protein nur in einem der beiden Reaktionsansätze enthalten ist.

Eine Ausführungsform des Verfahrens beinhaltet, daß
a) mindestens zwei Transkriptionen parallel unter gleichen Bedingungen durchgeführt werden, wobei
b) sich die Transkriptionsansätze nur dadurch unterscheiden, daß sie unterschiedliche Mengen des zu testenden Wirkstoffs und/oder mindestens eines Transkriptionsfaktors und/oder mindestens eines Kofaktors und/oder eines angereicherten Kernextrakts enthalten,
c) im Anschluß an die Transkription für jeden Ansatz die erhaltene Menge an markiertemTranskript bestimmt wird und
d) aus der Differenz der erhaltenen Mengen an markiertem Transkript die Wirksamkeit und/oder Spezifität des zu testenden Wirkstoffs, Transkriptionsfaktors, Kofaktors und/oder angereicherten Kernextrakts im Bezug auf das genregulatorische Element ermittelt wird.

Mit diesem Verfahren kann die Wirkung jeder einzelnen Komponente (z.B. auf den Kernextakt (d.h. auf einen bestimmten Zelltyp) oder einen bestimmten Transkriptionsfaktor), die im Transkriptionsansatz enthalten ist, gezielt getestet werden. Beispielsweise kann die Wirkung eines zu untersuchenden Wirkstoffs auf ein definiertes genregulatorisches Element analysiert werden. Entscheidend dabei ist, daß die Reaktionsbedingungen der Transkription exakt definiert werden können.

Das Verfahren bietet die Möglichkeit, die Wirkung einzelner Faktoren auf eine pathologische Genexpressionen gezielt zu beeinflussen, da entsprechende Reaktionsbedingungen durch die Auswahl des genregulatorischen Elements sowie der Transkriptions- und/ oder Kofaktoren und/oder zelltypspezifischen Kernextrakte genau eingestellt bzw. vorgegeben werden können.

Ein besonderer Vorteil des Verfahrens ist, daß auf diese Weise pharmakologische Wirkstoffe identifiziert werden können, die die Transkription unter definierten Bedingungen positiv oder negativ beinflussen können, insbesondere solche, die die Transkription definierter (Target-)Gene aktivieren oder inhibieren, wobei diese Wirkstoffe spezifische Wirkung auf definierte genregulatorische Elemente und/oder definierte Transkriptionsfaktoren, Kofaktoren und/oder zelltypspezifische Kernextrakte (bzw. Zellen) haben.

Die Erfindung betrifft ein Verfahren zur Identifizierung von spezifischen pharmakologisch aktiven Wirkstoffen. Beispielsweise kann das Verfahren verwendet werden, um einen zu testenden Wirkstoff unter definierten Bedingungen zu charakterisieren. Beispielsweise kann das Verfahren verwendet werden, um einen auf diese Weise identifizierten Wirkstoff in Bezug auf seine Spezifität unter definierten Bedingungen weiter zu charakterisieren. Beispielsweise sollte ein mit Hilfe des Verfahrens identifizierter pharmakologisch aktiver Wirkstoff unter definierten Bedingungen die Transkription der unter der Kontrolle des genregulatorischen Elements vorliegenden zu transkribierenden DNA-Sequenz inhibieren oder aktivieren.

Eine weitere besonders vorteilhafte Eigenschaft des Verfahrens ist, daß alle Schritte auf einfache Weise automatisiert werden können. Beispielsweise kann dafür der Pipettierroboter Biomek 2000® (Firma Beckman, München), angeschlossen an ein Versorgungsrobotermodul, eingesetzt werden. Die an eine feste Phase gebundenen Transkripte können dann manuell oder automatisch, z.B. mit Hilfe eines Fließbands gewaschen werden.

Die vorliegende Erfindung beinhaltet, daß ein Pipettierroboter, z.B. der Biomek 2000® mit den einzelnen Komponenten der Reaktion, wie Proteinfraktion (z.B. Kernextrakt und andere Proteine), DNA-Template, Transkriptionspuffer, in der Transkription zu untersuchende Substanzen, Pipettenspitzen, Mikrotiterplatten und Membranen bestückt wird. Aus diesen Komponenten werden die einzelnen Reaktionsansätze beispielsweise in den Vertiefungen von Mikrotiterplatten zusammengestellt, wobei alle Pipettierschritte automatisch erfolgen. Auf diese Weise können bei geringen Probenvolumina, insbesondere Volumina kleiner 100 µl, vorzugsweise von 10 bis 50 µl, insbesondere 20 µl, 96 oder mehr verschiedene Transkriptionsansätze pro Platte gleichzeitig bearbeitet werden. Jede Transkription dauert etwa 1 bis 1,5 Stunden, so daß bei optimaler Ausnutzung der Inkubationszeiten auf diese Weise bis zu 1000 oder mehr Transkriptionen pro Tag durchgeführt werden können.

Die Transkription kann beispielsweise bei Temperaturen von 20-50°C durchgeführt werden. Besonders bevorzugt ist die Durchführung der Transkription bei etwa 30°C.

Da insbesondere die zu testenden Wirkstoffe, Transkriptionsfaktoren, Kofaktoren und gegebenfalls auch die angereicherten Kernextrakte nur in geringen Mengen verfügbar sind, diese Substanzen aber unter verschiedensten Reaktionsbedingungen in einer Vielzahl von Transkriptionen getestet werden sollen, ist eine Anforderung an das Verfahren, daß für die Transkriptionsreaktion möglichst geringe Probenvolumina benötigt werden. Deshalb ist insbesondere von Bedeutung, daß dieses Verfahren auch für eine Durchführung im Nanolitermaßstab, d.h. Reaktionsvolumen von ca. 50-500 nl, geeignet ist.

Das Transkriptionsverfahren ist universell einsetzbar. Es kann zur Identifizierung und Charakterisierung von genregulatorischen Elementen (d.h. ein spezifisches Gen als Target), von Transkriptions- und/oder Kofaktoren und/oder anderen Proteinen, die direkt oder indirekt an der Regulation der Gentranskription beteiligt sind (d.h. ein spezifisches Protein als Target) und/oder von angereicherten Kernextrakten (d.h. ein spezifisches Kernextrakt bzw. ein spezifischer Zelltyp als Target) verwendet werden. Insbesondere kann das Transkriptionsverfahren zur Identifizierung neuer pathologisch interessanter genregulatorischer Elemente und der Zuordnung von genregulatorischen Proteinen, die die Wirkung dieser Elemente vermitteln, zu den entsprechenden genregulatorischen Elementen, benutzt werden.

Im Vergleich zu zellulären Assays weist das beschriebene Transkriptionsverfahren eine höhere Targetspezifität auf. Im Gegensatz zu zellulären Assays hat die zelluläre Aufnahme einzelner Komponenten keinen Effekt auf die Effizienz der Transkription. Darüber hinaus läßt sich das beschriebene Verfahren einfach und schnell durchführen (z.B. können die einzelnen Komponenten vorbereitet und gefroren aufbewahrt werden). Das Verfahren ist einfach zu standardisieren und universell einsetzbar, da es sich auf nahezu jeden Zelltyp und jedes Gen anwenden läßt.

Mit Hilfe des Verfahrens können pharmakologische Wirkstoffe identifiziert werden, die zur Herstellung von Arzneimittel verwendet werden können. Mit diesem Transkriptionsverfahren identifizierte Wirkstoffe sollten im Vergleich zu bekannten Wirkstoffen bedeutend weniger Nebenwirkungen hervorrufen. Beispielsweise können Wirkstoffe, die zur Herstellung von Arzneimitteln, zur Behandlung von (Auto-) Immunerkrankungen, Stoffwechselerkrankungen, Krebs, Herz-Kreislauferkrankungen, Infektionskrankheiten, Rheumatismus, Diabetis, degenerativen und psychischen Erkrankungen, insbesondere zur Herstellung von Arzneimitteln zur Behandlung von rheumatoider Arthritis, multipler Sklerose, Diabetis Mellitus, Allergien, Asthma, Anaphylaxe, atopischer Dermatitis, der Alzheimerschen Krankheit, der Parkinson Krankheit, AIDS, der Creutzfeldt-Jakobschen Krankheit, Epilepsie, Schizophrenie, Artheriosklerose und Tuberkulose eingesetzt werden können, getestet bzw. identifiziert werden.

Darüberhinaus bietet dieses universelle Verfahren eine Vielzahl weiterer Anwendungsmöglichkeiten. Das Verfahren kann z.B. auch in der Tierpathologie und Tierzüchtung, im Pflanzenschutz oder der Pflanzenzüchtung zur Auffindung von spezifischen, pharmakologischen Wirkstoffen analog eingesetzt werden, wenn die entsprechenden basalen Transkriptionssysteme aus den jeweiligen Organismen und die spezifischen genregulatorischen Elemente, Transkriptions- und/oder Kofaktoren und/oder andere direkt oder indirekt an der Transkriptionsreaktion beteiligten Proteine eingesetzt werden.

Prinzipiell läßt sich dieses in vitro Transkriptionsverfahren analog auch zur Identifizierung von Substanzen benutzen, die bei der Konservierung von Materialien und Lebensmitteln Verwendung finden können, wenn entsprechend die genregulatorischen Elemente, transkriptionsfähigen Systeme, Transkriptions- und/oder Kofaktoren von Mikroorganismen, beispielsweise von Hefen, Pilzen, Bakterien oder von Insekten verwendet werden.

Die Abbildungen sind wie folgt beschrieben:

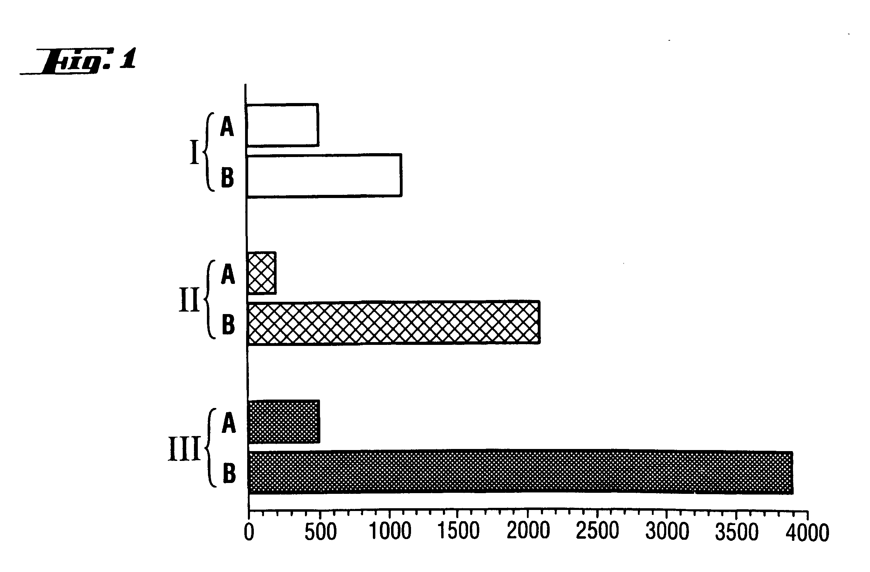

- Fig.1:: Radioaktiver "Read out" von Transkriptionsreaktionen, wobei verschiedene genregulatorische Elemente und Reporterplasmide, die G-freie Sequenzen unterschiedlicher Länge aufweisen, eingesetzt wurden.
Es ist der Vergleich zweier Standardpromotoren mit dem universellen Reporterplasmid pGS100 (Fig. 2) gezeigt. Die Transkriptionsreaktion wurde, wie in Beispiel 6 beschrieben, durchgeführt.
A) Basale Transkription: Menge an radioaktivem Transkript [in relativen Einheiten], die ohne Aktivierung des Promotors erhalten wird (basale Signalstärke);
B) Aktivierte Transkription: Menge an radioaktivem Transkript [in relativen Einheiten], die mit Aktivierung des Promotors mit Gal4-Polyglutamin erhalten wird.
I) Als Reporterplasmid wurde pMRG5 (Krezschmar, M., Kaiser, K., Lottspeich, F., Meisterernst, M. (1994) Cell 78, 525-534) verwendet. Der synthetische Promotor in pMRG5 enthält die TATA-Box des HIV Promotors, den Initiator des ML Promotors und eine etwa 400 Nukleotide lange G-freie Sequenz in pUC19.
   II) Als Reporterplasmid wurde pVβML verwendet. Das Reporterplasmid pVβML ist wie das Reporterplasmid pGS100 aufgebaut, enthält jedoch anstelle der 800 Nukleotide langen nur eine 400 Nukleotide lange G-freie Sequenz.
   III) Als Reporterplasmid wurde der pGS100 verwendet, der eine G-freie Sequenz von etwa 800 Nukleotiden aufweist.

Verglichen wurden jeweils die basale (A) mit der aktivierten Transkription (B). Als Aktivator wurde ein Fusionsprotein, bestehend aus einer Gal4-Bindungsdomäne (94 aminoterminale Aminosäuren) und einer Polyglutamin-Aktivierungsdomäne (synthetisches Peptid aus 11 Glutaminsäuren), eingesetzt. Es sind die Absolutwerte (in relativen Einheiten, wie sie mit Hilfe eines Phosphoimagers erhalten werden), nach Abzug des Hintergrunds, aufgetragen. Bei den Transkriptionsreaktionen, bei denen pGS100 als Reporterplasmid verwendet wurde, wurden sowohl höhere Absolutwerte, als auch, bedingt durch den unterschiedlichen Aufbau der synthetischen Promotoren, eine im Vergleich zu pMRG5 bessere Aktivierbarkeit gemessen. Darüber hinaus wird der positive Einfluß einer G-freien Sequenz, die eine Länge von mehr als 400 Nukleotiden aufweist, deutlich.
- Fig. 2:: Universelles Reporterplasmid pGS100.
Das universelle Reporterplasmid pGS100 enthält einen synthetischen Promotorbereich als Modellpromotor vor einer etwa 800 Basenpaare langen G-freien Region in pUC19. Innerhalb der Restriktionsschnittstellen BamHI und Swal befindet sich der Initiatorbereich des adenoviralen "major late" (ML) Promotors. Zwischen den Restriktionsschnittstellen für SacII und BamHI befindet sich der Bereich mit TATA-Box des humanen T-Zellrezeptor Vβ 8.1 Promotors. Diese beiden basalen Promotorelemente (Initiator und TATA Box) ermöglichen die basale Transkription in vitro. Da diese in potentiellen Targetgenen für das Screening von spezifischer Bedeutung sein können, ist es möglich, diese einzeln gegen die entsprechenden Bereiche der zu untersuchenden Gene (entsprechende genregulatorische Elemente) auszutauschen. In den Polylinker stromabwärts des basalen Promotors können beliebige regulatorische Bereiche von Zielgenen eingefügt werden (spezifische Aktivierung). Alternativ kann der gesamte Promotorbereich ausgetauscht werden. Stromaufwärts des Polylinkers (Sac II bis Pstl) enthält pGS100 fünf Bindungsstellen für das Hefe Gal4 Protein. Dieses ermöglicht auch die Analyse von synthetischen Transkriptionsaktivatoren, beispielsweise von Fusionsproteinen, die aus einer beliebigen Aktivierungsdomäne (z.B. des Herpes Simplex Transaktivators VP16) und einer Gal4 DNA Bindungsdomäne bestehen. Ein essentieller Baustein von pGS100 ist die G-freie Sequenz.
- Fig. 3:: Vergleich der Effizienz eines herkömmlichen Standard-Transkriptionsverfahrens mit dem beschriebenen zellfreien in vitro Transkriptionsverfahren.

Fig. 3 zeigt einen Vergleich der Signalstärken (als Maß für die Menge an Transkript und damit für die Stärke der Trankription), die unter unterschiedlichen Reaktionsbedingungen erhalten werden. Die Transkriptionsreaktionen werden wie in Beispiel 7 beschrieben durchgeführt.
A) Herkömmliches Standard-Transkriptionsverfahren, bei dem die spezifischen Transkripte durch Phenolisierung, Ethanolfällung und anschließende denaturierende Gelelektrophorese von Proteinen und überschüssigen Nukleotiden befreit werden;
B) beschriebenes in vitro Transkriptionsverfahren, bei dem die Proteine durch Proteinase K verdaut und die überschüssigen Nukleotide durch Waschen der an DEAE-Filter gebundenen spezifischen Transkripte entfernt werden;
   l) für die Transkription wird ein transkriptionsfähiges, über eine P11®-Säule gereinigtes Extrakt aus HeLa-Zellkernen, ohne entsprechendes DNA-Template eingesetzt (Kontrollexperiment);
   II) Basale Transkription: zu einem dem Ansatz aus I entsprechenden Ansatz wird als DNA-Template das Reporterplasmid pMRG5 gegeben und die Transkriptionsreaktion durchgeführt; auf diese Weise wird die basale Signalstärke bestimmt;
   III) Aktivierte Transkription: zu einem dem Ansatz II entsprechenden Ansatz wird zusätzlich der Transkriptionsaktivator, ein Fusionsprotein, bestehend aus der DNA-Bindungsdomäne von Gal4 und der Aktivierungsdomäne von VP16 (Gal4-VP16), gegeben und die Transkription durchgeführt; auf diese Weise wird die aktivierte Signalstärke erhalten;
   IV) zu einem dem Transkriptionsansatz aus III entsprechenden Ansatz, wurde α-Amanitin als RNA-Polymerase II Hemmer zugegeben (Kontrollexperiment).

Ein Vergleich der Signalstärken, die unter den unterschiedlichen Reaktionsbedingungen mit den zwei verschiedenen Verfahren erhalten wurden zeigt, daß sowohl die basale (II) als auch die aktivierte (III) Transkription mit dem hier beschriebenen in vitro Transkriptionsverfahren meßbar sind, wobei die Signalstärken mit denen, die mit einem herkömmlichen Transkriptionsverfahren erhalten werden, vergleichbar sind.
Daß auch die basale Signalstärke meßbar ist, ist von entscheidender Bedeutung, damit das beschriebene Transkriptionsverfahren auch zum Screening von Transkriptionsinhibitoren eingesetzt werden kann. Mit Hilfe des spezifischen RNA Polymerase II Inhibitors α-Amanitin wird eine etwa 8-fache Abnahme des Signals (Vergleich III und IV) erzielt.

### Beispiele:

### Beispiel 1: Herstellung von HeLa-Kernextrakten

Die Kernextrakte werden ausgehend von HeLa-Zellkernen hergestellt. HeLa-Zellkerne sind bei verschiedenen Firmen (z.B "4°C", Mons; Sigma, München; Santa Cruz Biotechnology, Heidelberg) käuflich zu erwerben. Die im folgenden beschriebene Aufarbeitung der Zellkerne erfolgt bei 4 °C (Kühlraum). Die Puffer werden mit Tris pH 6.8 bei Raumtemperatur (RT) eingestellt, dies entspricht pH 7.3 bei 4 °C. Den Puffern wird vor Gebrauch DTT (Stammlösung 1 M in Wasser) zur Endkonzentration von 5 mM und PMSF (Stammlösung 200 mM in DMSO) zur Endkonzentration von 1 mM zugesetzt.

Die Aufarbeitung der Zellkerne umfaßt folgende Schritte:
1. Zellkerne auf Eis auftauen und Volumen NPV (nuclear pellet volume) bestimmen.
2. Jeweils 1/2 NPV-Volumen an 0,02 M KCI Puffer (Puffer mit niedrigem Salzgehalt: 20 ml 1 M Tris pH 6.8 RT, 250 ml 100 % Glycerin, 6,67 ml 3 M KCI, 1,5 ml 1 M MgCl_{2,} 0,4 ml 0,5 M EDTA, H₂O ad 1000 ml) und 1,2 M KCI Puffer (Puffer mit hohem Salzgehalt: 20 ml 1 M Tris pH 6.8 RT, 250 ml 100 % Glycerin, 400 ml 3 M KCI, 1,5 ml 1 M MgCl₂, 0,4 ml 0,5 M EDTA, H₂O ad 1000 ml) werden in 2 separate Bechergläser geben.
   Zu jedem Puffer werden 0,0007 x NPV/2 β-Mercaptoethanol und 0,001 x NPV/2 0,2 M PMSF gegeben.
   Das Pellet wird in 1/2 Volumen 0,02 M KCl Puffer resuspendiert und sanft mit einem Pistill homogenisiert (6 x).
3. Homogenat in Becherglas vorlegen und tropfenweise über 30 min mit 1,2 M KCI Puffer unter ständigem Rühren versetzen. Die Extraktion ist nach weiteren 30 min Rühren beendet. Abzentrifugieren (Beckman-Zentrifuge, SS34-Rotor bei 14000 rpm, 30 min, 4 °C), Pellet und Überstand getrennt weiter verarbeiten.
4. Überstand in Puffer 1 (40 ml 1 M Tris pH 6.8 RT, 400 ml Glycerin, 0,8 ml 0,5 M EDTA, H₂O ad 2000 ml) dialysieren, bis die Leitfähigkeit von Puffer 2 (40 ml 1 M Tris pH 6.8 RT, 400 ml Glycerin, 0,8 ml 0,5 M EDTA, 66,7 ml 3 M KCI, H₂O ad 2000 ml) erreicht ist (45 - 55 min).
5. Dialysierten Überstand abzentrifugieren (Beckman, SS34-Rotor, 18000 rpm, 20 min, 4 °C). Der HeLa-Kernextrakt befindet sich im Überstand (HeLa Nuclear Extrakt = HeLa NE). Aliquots des Extraktes werden in flüssigem N₂ eingefroren. Pellet aus der Kernextraktion in Homogenisator transferrieren, mit 10 ml TGME/5 mM DTT (TGME für 1I: 250 ml 100 % Glycerin, 50 ml Tris pH 7.3 RT, 5 ml 1 M MgCl₂, 0,2 ml 500 mM EDTA pH 8,0) versetzen, 20 x mit einemPistill homogenisieren (kräftig) und in flüssigem N₂ einfrieren (HeLa Nuclear Pellet).

### Beispiel 2: Vorbereitung der Phosphocellulosesäule für die Aufreinigung des Kernextrakts.

1. P11® Säulenmaterial mehrmals in Wasser waschen. Volumen des gequollenen Materials bestimmen.
2. 5 Volumen 0,5 N NaOH zugeben, 5 min stehen lassen, dann sofort über Faltenfilter absaugen.
3. Waschen mit Wasser bis pH 11.
4. 25 Volumen 0,5 N HCl zugeben. 5 min stehen lassen, dann sofort absaugen.
5. Waschen mit Wasser bis pH 3.
6. Waschen mit 1 M Tris pH 7 bis pH konstant bei 7. Säulenmaterial bei 4 °C aufbewahren. Am besten über Nacht äquilibrieren.

### Beispiel 3: Chromatographische Reinigung des Kernextraktes durch eine Phosphocellulose Chromatographie:

Die Kapazität des P11® Materials beträgt 10 mg Protein/1 ml Material. Die Chromatographie wird wie folgt durchgeführt:
1. P11® Material in Säule überführen und in Puffer 2 (mit frisch zugegebenem DTT und PMSF, siehe Beispiel 1) äquilibrieren. Säule an Pumpe anschließen.
2. Kernextrakt (in Puffer 2) beladen (mit 1 Säulenvolumen (column volume) (CV)/h).
3. Säule waschen mit 5 CV Puffer 2 (5 - 10 CV/h).
4. Eluieren mit Puffer 3 (40 ml 1 M Tris pH 6.8 RT, 400 ml Glycerin, 0,8 ml 0,5 M EDTA, 200 ml 3 M KCI, H₂O ad 2000 ml; 2 CV/h). Nachdem kein Protein mehr eluiert, weitere 2 CV Puffer 3 durchlaufen lassen.
5. Eluieren mit Puffer 4 (40 ml 1 M Tris pH 6.8 RT, 400 ml Glycerin, 0,8 ml 0,5 M EDTA, 333 ml 3 M KCl, H₂O ad 2000 ml; 2 CV/h) und Peakfraktionen sammeln.
6. Eluieren mit Puffer 5 (40 ml 1 M Tris pH 6.8 RT, 400 ml Glycerin, 0,8 ml 0,5 M EDTA, 567 ml 3 M KCI, H₂O ad 2000 ml; 2 CV/h) und Peakfraktionen sammeln.
7. Die beiden Eluate jeweils gegen Puffer 2 dialysieren, bis Leitfähigkeit konstant ist und dann Aliquots in flüssigem N₂ einfrieren.

### Beispiel 4: Standard-Transkriptionsverfahren unter Verwendung eines Polyacrylamidgels zur Auftrennung der Transkripte.

Ein Transkriptionsansatz (20 µl Endvolumen) kann aus folgenden Komponenten bestehen:
a) generelle Transkriptionsfaktoren in Form von vorgereinigten Kernextrakten oder rekombinant hergestellt) eventuell ergänzt durch weitere spezifische Transkriptionsfaktoren, Aktivatoren, Inhibitoren oder Fusionsproteine;
b) DNA-Template (z.B. pGS100 Vektor mit zu untersuchendem genregulatorischem Element);
c) Transkriptionspuffer, der beispielweise den zu untersuchenden Wirkstoff enthält;
c) markierte und unmarkierte Nukleotide;
   zu a) Puffer 4-Eluat und Puffer 5-Eluat (aus Beispiel 3) enthält das transkriptionsfähige Kernextrakt (alle generellen Transkriptionsfaktoren). Die optimalen Mengen an Puffer 4-Eluat und Puffer 5-Eluat müssen für jede Präparation neu bestimmt werden (ca. 3 µl Puffer 4-Eluat und 2 µl Puffer 5-Eluat pro Ansatz);
   zu b) gewöhnlich werden 100 ng DNA-Template (genregulatorisches Element im Reporterplasmid z.B. in pGS100) pro Ansatz eingesetzt;
   zu c) Transkriptionspuffer (5 mM MgCl₂, 25 mM HEPES KOH pH 8.2, 0,5 µl BSA acetyliert (Stock 20 mg/ml), ca. 10 % Glycerin, ca. 70 mM KCl, 0,2 mM PMSF, 10 mM DTT) mit je 20 U RNase Inhibitor pro Ansatz;
   zu d) NTPs (ATP, UTP je 100 µM Endkonzentration, CTP 5 µM Endkonz., o-m-GTP 20 µM Endkonzentration, α-³²P-CTP ca. 0,12 µM Endkonzentration 3000 Ci/mmol, 10 mCi/ml).
      1. Der Transkriptionspuffer und dNTPs werden vorlegt und das DNA-Template zugegeben.
      2. Transkriptionsaktivator und Puffer 4- und 5-Eluat werden zugeben.
      3. Zur Durchführung der Transkriptionsreaktion 1 h bei 30 °C inkubieren.
      4. Zugabe von 400 µl Stopmix (7M Harnstoff, 10 mM Tris HCl pH 7,8, 10 mM EDTA/NaOH pH 8, 0,5 % SDS, 100 mM LiCl, 100 µg/ml tRNA, 300 mM NaAcetat) und 400 µl Phenol/Chloroform/Isoamylalkohol (25/24/1). Mischen und zentrifugieren (SS34-Rotor, Beckman Zentrifuge, 5 min, 14000 rpm, RT).
         Überstand abnehmen und 400 µl Isopropanol zugeben und mischen, dann 1 h bei - 20 °C inkubieren.
      5. Zentrifugieren (SS34 Rotor, Beckman Zentrifuge, 14000 rpm, 30 min, 4 °C), Pellet in 70 % Ethanol waschen und dann in der Speedvac trocknen.
      6. Pellet in 10 µl Auftragspuffer (955 µl 100 % Formamid (deionisiert), 10 µl 0,5 M EDTA, 20 µl 1M Tris pH 7, je 0,003 % Bromphenolblau/Xylencyanol, H₂O ad 1 ml) aufnehmen und 15 min bei 50 °C inkubieren.
      7. Auftrennung der Transkripte im 5 %igen denaturierenden Harnstoff-Polyacrylamidgel mit 1 x TBE als Laufpuffer. Vorlauf 20 min bei 60 mA. Gel laden. Gellauf ca. 1 h bei 60 mA.
      8. Gel in 10 % Essigsäure fixieren, trocknen und einem Röntgenfilm in einem Phosphoimager® exponieren.

### Beispiel 5: Protokoll des beschriebenen automatisierbaren in vitro Transkriptionsverfahrens unter Verwendung eines Filters zur Bindung der Transkripte

1. Der Transkriptionspuffer und dNTPs werden vorlegt und das DNA-Template zugegeben.
2. Puffer 4- und 5-Eluat und eventuell Transkriptionsaktivator oder Transkriptionsinhibitor werden zugeben.
3. Zur Durchführung der Transkriptionsreaktion wird 1 h bei 30 °C inkubiert.
4. Zugabe von 5 µl Proteinase K Mix (1 µl Proteinase K-Lösung [20 mg/ml], 1 µl 10 % SDS, 0,5 µl 0,5 M EDTA pH 8, 1 µl 50 mM Tris pH 7,8, H₂O ad 5 µl).
5. Inkubation 15 min 30 °C.
6. DEAE Membran NA 45® (Schleicher und Schuell) kurz in Membranwaschpuffer (100 mM Natriumphosphatpuffer pH 7,5, 250 mM NaCl, 2 % Natriumpyrophosphat) waschen.
7. Von dem Ansatz 5 µl auf die Membran pipettieren und 5 min trocknen lassen.
8. Membran 4 x 15 min in ca. 100 ml Membranwaschpuffer (+ 1 % Triton) leicht schütteln.
9. Membran auf Whatmann 3 MM® Papier (Firma Whatman, Maidstone, England) überführen, an der Luft trocknen lassen, Folie überziehen und die Filter unter Verwendung eines Phosphoimagers® einem Röntgenfilm exponieren.

### Beispiel 6: Die Transkriptionsreaktion wird nach dem beschriebenen in vitro Transkriptionsverfahren unter Verwendung eines Filters parallel mit drei verschiedenen Reporterplasmiden durchgeführt.

Die Durchführung erfolgt wie in den Beispielen 1 bis 3 und 5 beschrieben. Als Reporterplasmide werden der pMRG5 (TATA-Box des HIV Promotors, Initiatorregion des ML Promotors und eine etwa 400 Nukleotide lange G-freie Kassette in pUC19 (Krezschmar, M., Kaiser, K, Lottspeich, F., Meisterernst, M. (1994) Cell. 78, 525-534), der pGS100 (Fig. 2) und der pVβML (wie pGS100 aufgebaut, enthält jedoch anstelle der 800 bp langen nur eine 400 bp lange G-freie Kassette) verwendet. Für die Transkriptionsreaktionen werden jeweils 100 ng DNA-Template und 3 µl der P11®-Fraktionen (Puffer 4 und Puffer 5 Eluat) eingesetzt. Die Reaktionen werden jeweils parallel ohne und mit Aktivator (Fusionsprotein bestehend aus Gal4 Bindungsdomäne und einer Polyglutamin Aktivierungsdomäne) durchgeführt. Die Ergebnisse sind in Fig. 1 dargestellt und erläutert.

### Beispiel 7: Vergleich der Signalstärke (als Maß für die Menge an Transkript und damit für die Stärke der Trankription) der radioaktiv markierten Transkripte erhalten mit einem Standard-Transkriptionsverfahren mit denen, die mit dem beschriebenen in vitro Transkriptionsverfahren erhalten werden.

Die Transkriptionsreaktionen werden wie in den Beispielen 1 bis 5 beschrieben durchgeführt und parallele Reaktionsansätze im Filter- (Beispiel 5) beziehungsweise Gelassay (Beispiel 4) analysiert. Für die Reaktionen werden jeweils 200 ng pMRG5 als DNA-Template eingesetzt. Einige Transkriptionsreaktionen werden in Gegenwart von 30 ng Gal4-VP16, das als Aktivator eingesetzt wird, durchgeführt. Die Ergebnisse sind in Fig. 3 dargestellt und beschrieben.

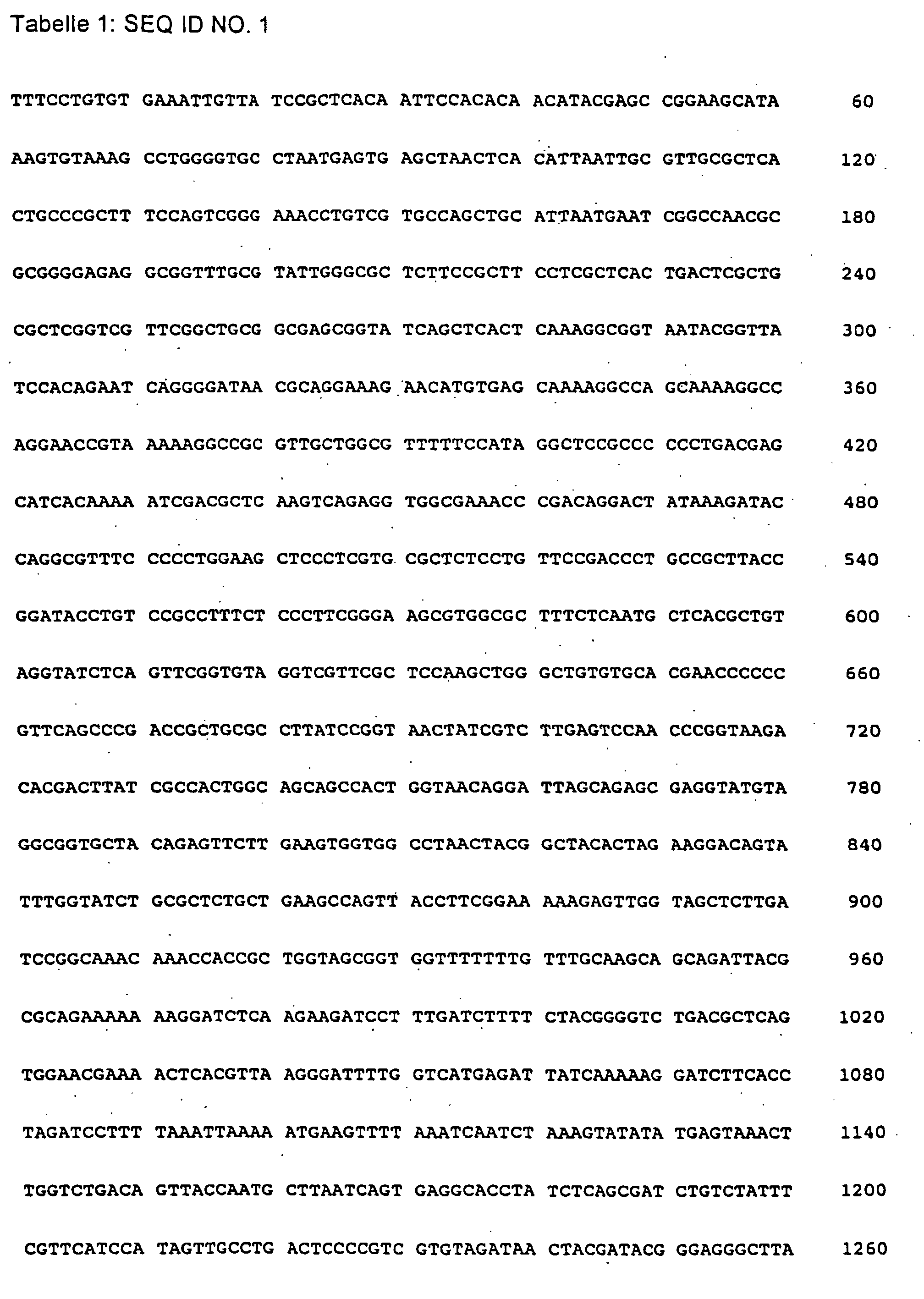

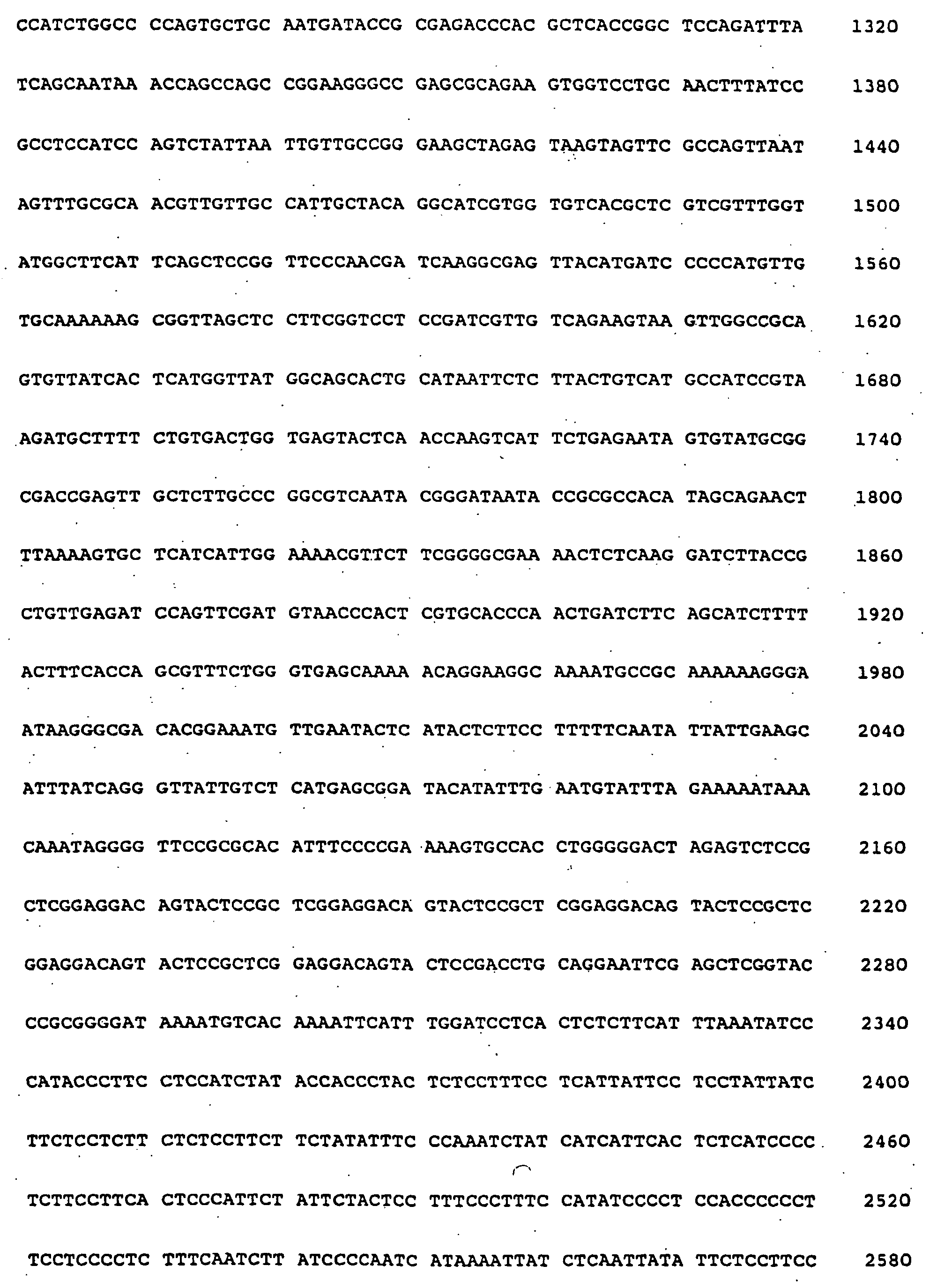

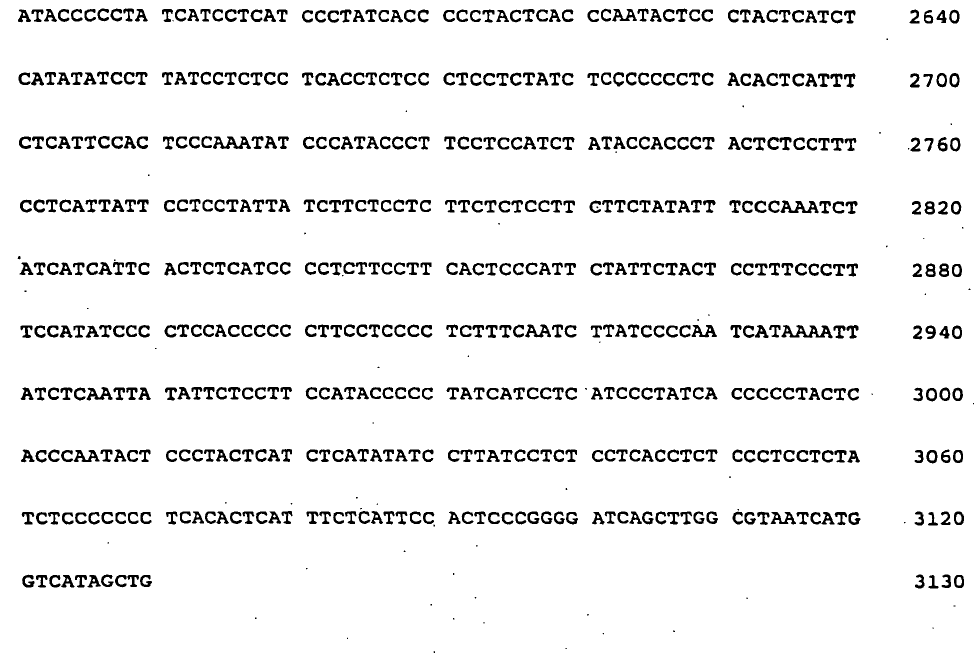

## Patentansprüche

1. Verfahren zur Identifizierung von pharmakologisch aktiven Wirkstoffen umfassend die zellfreie in vitro Transkription umfassend
a) Bereitstellung eines DNA-Templates, enthaltend eine zu transkribierende DNA-Sequenz, die unter der Kontrolle mindestens eines genregulatorischen Elementes vorliegt, Mischung mit mindestens einem markierten Nukleotid einem Kernextrakt und eines zu testenden Wirkstoffs und Durchführung der Transkriptionsreaktion.
b) Abtrennung und/oder Degradierung der im Ansatz enthaltenen Proteine im Anschluss an die Transkription.
c) Bindung des markierten Transkriptes, an einen festen Träger.
d) Entfernung der überschüssigen markierten Nukleotide.
e) Bestimmung der Menge markierten Transkriptes relativ zu einem parallelen Ansatz in Abwesenheit des zu testenden Wirkstoffes wobei das DNA Template singuläre Schnittstellen für die Restriktionsendonukleasen PstI, EcoRI, Sacl, Kpnl, SacII, BamHI, Swal und Smal enthält, wobei fünf Bindungsstellen für das Hefe Gal4 Protein und die "TATA"-Box des humanen T-Zellrezeptors Vβ 8.1 zwischen den Restriktionsschnittstellen für SacII und BamHI, die INR (Initiator)-Region des ML-Promotors (adenovirus major late promoter) zwischen den Restriktionsschnittstellen BamHI und Swal und eine G-freie Sequenz mit einer Länge von etwa 800 Nukleotiden (Basenpaaren) zwischen den Restriktionsschnittstellen Swal und Smal in das pUC19 Plasmid kloniert sind.

2. Verfahren nach Anspruch 1, wobei das universelle Reporterplasmid die Sequenz gemäß SEQ ID NO. 1 aufweist.

3. Verfahren nach Anspruch 1, wobei das DNA-Template das universelle Reporterplasmid ist, welches unter der DSM-Nummer 11450 hinterlegt wurde.

4. DNA-Template enthaltend singuläre Schnittstellen für die Restriktionsendonukleasen Pstl, EcoRI, Sacl, Kpnl, Sacll, BamHI, Swal, SmaI, fünf Bindungsstellen für das Hefe Gal4 Protein, die "TATA"-Box des humanen T-Zellrezeptors Vβ 8.1 zwischen den Restriktionsschnittstellen für SacII und BamHI, die INR (Initiator)-Region des ML-Promotors (adenovirus major late promoter) zwischen den Restriktionsschnittstellen BamHI und Swal und eine G-freie Sequenz mit einer Länge von etwa 800 Nukleotiden (Basenpaaren) in dem pUC19 Plasmid.

5. DNA-Template nach Anspruch 4 mit der SEQ ID NO. 1.

6. DNA-Template nach Anspruch 4, welches unter der DSM-Nummer 11450 hinterlegt ist.

## Claims

1. Method for identifying pharmacologically active ingredients comprising the cell-free *in-vitro* transcription comprising
a) providing a DNA template comprising a DNA sequence to be transcribed which is present under the control of at least one gene-regulatory element, mixing with at least one labelled nucleotide, a nuclear extract and an active ingredient to be tested, and carrying out the transcription reaction,
b) subsequently to the transcription, removing and/or degrading of the proteins present in the mixture,
c) binding the labelled transcript to a solid support,
d) removing of the excess labelled nucleotides,
e) determining of the amount of labelled transcript in relation to a parallel mixture in the absence of the active ingredient to be tested, the DNA template comprising singular cleavage sites for the restriction endonucleases PstI, EcoRI, SacI, KpnI, SacII, BamHI, SwaI and SmaI, five binding sites for the yeast Gal4 protein and the "TATA" box of the human T-cell receptor Vβ 8.1 being cloned into the plasmid pUC19 between the restriction cleavage sites for SacII and BamHI, the INR (initiator) region of the ML promoter (adenovirus major late promoter) being cloned into the plasmid pUC19 between the restriction cleavage sites BamHI and SwaI, and a G-free sequence with a length of approximately 800 nucleotides (base pairs) being cloned into the plasmid pUC19 between the restriction cleavage sites SwaI and SmaI.

2. Method according to Claim 1, the universal reporter plasmid having the sequence as shown in SEQ ID No. 1.

3. Method according to Claim 1, the DNA template being the universal reporter plasmid which has been deposited as DSM number 11450.

4. DNA template comprising singular cleavage sites for the restriction endonucleases PstI, EcoRI, SacI, KpnI, SacII, BamHI, SwaI, SmaI, five binding sites for the yeast Gal4 protein, the "TATA" box of the human T-cell receptor Vβ 8.1 between the restriction cleavage sites for SacII and BamHI, the INR (initiator) region of the ML promoter (adenovirus major late promoter) between the restriction cleavage sites BamHI and SwaI, and a G-free sequence with a length of approximately 800 nucleotides (base pairs) in the plasmid pUC19.

5. DNA template according to Claim 4, having the SEQ ID No. 1.

6. DNA template according to Claim 4, which has been deposited as DSM number 11450.

## Revendications

1. Procédé pour l'identification de principes pharmacologiquement actifs, comprenant la transcription in vitro sans cellules comprenant :
a) la préparation d'une matrice d'ADN contenant une séquence d'ADN à transcrire, qui est placée sous le contrôle d'au moins un élément régulateur de gène, le mélange avec au moins un nucléotide marqué, un extrait de noyau et un principe actif à tester et la réalisation de la réaction de transcription,
b) la séparation et/ou la dégradation des protéines contenues dans la fraction à la suite de la transcription,
c) la liaison du transcrit marqué sur un support solide,
d) l'élimination des nucléotides marqués excédentaires,
e) la détermination de la quantité de transcrit marqué par rapport à une fraction parallèle en l'absence du principe actif à tester, dans lequel procédé la matrice d'ADN contient des sites de coupure singuliers pour les endonucléases de restriction PstI, EcoRI, SacI, KpnI, SacII, BamHI, SwaI et SmaI, et dans lequel on a cloné cinq sites de liaison pour la protéine de levure Gal4 et la boîte "TATA" du récepteur humain Vβ 8.1 de cellules T entre les sites de coupure de restriction SacII et BamHI, la région (initiatrice) RIN du promoteur ML (promoteur majeur tardif de l'adénovirus) entre les sites de coupure de restriction BamHI et SwaI et une séquence exempte de G d'une longueur d'environ 800 nucléotides (paires de bases) entre les sites de coupure de restriction SwaI et SmaI, dans le plasmide pUC19.

2. Procédé selon la revendication 1, dans lequel le plasmide rapporteur universel présente la séquence selon SEQ ID NO. 1.

3. Procédé selon la revendication 1, dans lequel la matrice d'ADN est le plasmide rapporteur universel, qui est déposé sous le numéro DSM 11450.

4. Matrice d'ADN contenant des sites de coupure singuliers pour les endonucléases de restriction PstI, EcoRI, SacI, KpnI, SacII, BamHI, SwaI, SmaI, cinq sites de liaison pour la protéine de levure Gal4 et la boîte "TATA" du récepteur humain Vβ 8.1 de cellules T entre les sites de coupure de restriction SacII et BamHI, la région (initiatrice) RIN du promoteur ML (promoteur majeur tardif de l'adénovirus) entre les sites de coupure de restriction BamHI et SwaI et une séquence exempte de G d'une longueur d'environ 800 nucléotides (paires de bases) dans le plasmide pUC19.

5. Matrice d'ADN selon la revendication 4 de SEQ ID NO. 1.

6. Matrice d'ADN selon la revendication 4, qui est déposée sous le numéro DSM 11450.


### SEQUENZPROTOKOLL

(1) ALLGEMEINE INFORMATION:
   (i) ANMELDER:
      (A) NAME: Hoechst Aktiengesellschaft
      (B) STRASSE: -
      (C) ORT: Frankfurt
      (D) BUNDESLAND: -
      (E) LAND: Deutschland
      (F) POSTLEITZAHL: 65926
      (G) TELEPHON: 069-305-7072
      (H) TELEFAX: 069-35-7175
      (I) TELEX: -
   (ii) ANMELDETITEL: In vitro Transkriptionsverfahren zum Screening von Naturstoffen und anderen chemischen Substanzen
   (iii) ANZAHL DER SEQUENZEN: 1
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 3130 Basenpaare
      (B) ART: Nukleinsäure
      (C) STRANGFORM: Doppel
      (D) TOPOLOGIE: ringförmig
   (ii) ART DES MOLEKÜLS: DNS (genomisch)
   (ix) MERKMALE:
      (A) NAME/SCHLÜSSEL: exon
      (B) LAGE: 1..3130.
   (xi) SEQUENZBESCHREIBUNG: SEQ ID NO: 1:
   TTTCCTGTGT GAAATTGTTA TCCGCTCACA ATTCCACACA ACATACGAGC CGGAAGCATA      60
   AAGTGTAAAG CCTGGGGTGC CTAATGAGTG AGCTAACTCA CATTAATTGC GTTGCGCTCA     120
   CTGCCCGCTT TCCAGTCGGG AAACCTGTCG TGCCAGCTGC ATTAATGAAT CGGCCAACGC     180
   GCGGGGAGAG GCGGTTTGCG TATTGGGCGC TCTTCCGCTT CCTCGCTCAC TGACTCGCTG     240
   CGCTCGGTCG TTCGGCTGCG GCGAGCGGTA TCAGCTCACT CAAAGGCGGT AATACGGTTA     300
   TCCACAGAAT CAGGGGATAA CGCAGGAAAG AACATGTGAG CAAAAGGCCA GCAAAAGGCC     360
   AGGAACCGTA AAAAGGCCGC GTTGCTGGCG TTTTTCCATA GGCTCCGCCC CCCTGACGAG     420
   CATCACAAAA ATCGACGCTC AAGTCAGAGG TGGCGAAACC CGACAGGACT ATAAAGATAC     480
   CAGGCGTTTC CCCCTGGAAG CTCCCTCGTG CGCTCTCCTG TTCCGACCCT GCCGCTTACC     540
   GGATACCTGT CCGCCTTTCT CCCTTCGGGA AGCGTGGCGC TTTCTCAATG CTCACGCTGT     600
   AGGTATCTCA GTTCGGTGTA GGTCGTTCGC TCCAAGCTGG GCTGTGTGCA CGAACCCCCC     660
   GTTCAGCCCG ACCGCTGCGC CTTATCCGGT AACTATCGTC TTGAGTCCAA CCCGGTAAGA 720
   CACGACTTAT CGCCACTGGC AGCAGCCACT GGTAACAGGA TTAGCAGAGC GAGGTATGTA 780
   GGCGGTGCTA CAGAGTTCTT GAAGTGGTGG CCTAACTACG GCTACACTAG AAGGACAGTA 840
   TTTGGTATCT GCGCTCTGCT GAAGCCAGTT ACCTTCGGAA AAAGAGTTGG TAGCTCTTGA 900
   TCCGGCAAAC AAACCACCGC TGGTAGCGGT GGTTTTTTTG TTTGCAAGCA GCAGATTACG 960
   CGCAGAAAAA AAGGATCTCA AGAAGATCCT TTGATCTTTT CTACGGGGTC TGACGCTCAG 1020
   TGGAACGAAA ACTCACGTTA AGGGATTTTG GTCATGAGAT TATCAAAAAG GATCTTCACC 1080
   TAGATCCTTT TAAATTAAAA ATGAAGTTTT AAATCAATCT AAAGTATATA TGAGTAAACT 1140
   TGGTCTGACA GTTACCAATG CTTAATCAGT GAGGCACCTA TCTCAGCGAT CTGTCTATTT 1200
   CGTTCATCCA TAGTTGCCTG ACTCCCCGTC GTGTAGATAA CTACGATACG GGAGGGCTTA 1260
   CCATCTGGCC CCAGTGCTGC AATGATACCG CGAGACCCAC GCTCACCGGC TCCAGATTTA 1320
   TCAGCAATAA ACCAGCCAGC CGGAAGGGCC GAGCGCAGAA GTGGTCCTGC AACTTTATCC 1380
   GCCTCCATCC AGTCTATTAA TTGTTGCCGG GAAGCTAGAG TAAGTAGTTC GCCAGTTAAT 1440
   AGTTTGCGCA ACGTTGTTGC CATTGCTACA GGCATCGTGG TGTCACGCTC GTCGTTTGGT 1500
   ATGGCTTCAT TCAGCTCCGG TTCCCAACGA TCAAGGCGAG TTACATGATC CCCCATGTTG 1560
   TGCAAAAAAG CGGTTAGCTC CTTCGGTCCT CCGATCGTTG TCAGAAGTAA GTTGGCCGCA 1620
   GTGTTATCAC TCATGGTTAT GGCAGCACTG CATAATTCTC TTACTGTCAT GCCATCCGTA 1680
   AGATGCTTTT CTGTGACTGG TGAGTACTCA ACCAAGTCAT TCTGAGAATA GTGTATGCGG 1740
   CGACCGAGTT GCTCTTGCCC GGCGTCAATA CGGGATAATA CCGCGCCACA TAGCAGAACT 1800
   TTAAAAGTGC TCATCATTGG AAAACGTTCT TCGGGGCGAA AACTCTCAAG GATCTTACCG 1860
   CTGTTGAGAT CCAGTTCGAT GTAACCCACT CGTGCACCCA ACTGATCTTC AGCATCTTTT 1920
   ACTTTCACCA GCGTTTCTGG GTGAGCAAAA ACAGGAAGGC AAAATGCCGC AAAAAAGGGA 1980
   ATAAGGGCGA CACGGAAATG TTGAATACTC ATACTCTTCC TTTTTCAATA TTATTGAAGC 2040
   ATTTATCAGG GTTATTGTCT CATGAGCGGA TACATATTTG AATGTATTTA GAAAAATAAA 2100
   CAAATAGGGG TTCCGCGCAC ATTTCCCCGA AAAGTGCCAC CTGGGGGACT AGAGTCTCCG 2160
   CTCGGAGGAC AGTACTCCGC TCGGAGGACA GTACTCCGCT CGGAGGACAG TACTCCGCTC 2220
   GGAGGACAGT ACTCCGCTCG GAGGACAGTA CTCCGACCTG CAGGAATTCG AGCTCGGTAC 2280
   CCGCGGGGAT AAAATGTCAC AAAATTCATT TGGATCCTCA CTCTCTTCAT TTAAATATCC 2340
   CATACCCTTC CTCCATCTAT ACCACCCTAC TCTCCTTTCC TCATTATTCC TCCTATTATC 2400
   TTCTCCTCTT CTCTCCTTCT TCTATATTTC CCAAATCTAT CATCATTCAC TCTCATCCCC 2460
   TCTTCCTTCA CTCCCATTCT ATTCTACTCC TTTCCCTTTC CATATCCCCT CCACCCCCCT 2520
   TCCTCCCCTC TTTCAATCTT ATCCCCAATC ATAAAATTAT CTCAATTATA TTCTCCTTCC 2580
   ATACCCCCTA TCATCCTCAT CCCTATCACC CCCTACTCAC CCAATACTCC CTACTCATCT 2640
   CATATATCCT TATCCTCTCC TCACCTCTCC CTCCTCTATC TCCCCCCCTC ACACTCATTT 2700
   CTCATTCCAC TCCCAAATAT CCCATACCCT TCCTCCATCT ATACCACCCT ACTCTCCTTT    2760
   CCTCATTATT CCTCCTATTA TCTTCTCCTC TTCTCTCCTT CTTCTATATT TCCCAAATCT    2820
   ATCATCATTC ACTCTCATCC CCTCTTCCTT CACTCCCATT CTATTCTACT CCTTTCCCTT    2880
   TCCATATCCC CTCCACCCCC CTTCCTCCCC TCTTTCAATC TTATCCCCAA TCATAAAATT    2940
   ATCTCAATTA TATTCTCCTT CCATACCCCC TATCATCCTC ATCCCTATCA CCCCCTACTC    3000
   ACCCAATACT CCCTACTCAT CTCATATATC CTTATCCTCT CCTCACCTCT CCCTCCTCTA    3060
   TCTCCCCCCC TCACACTCAT TTCTCATTCC ACTCCCGGGG ATCAGCTTGG CGTAATCATG    3120
   GTCATAGCTG                                                          3130